# EUROPEAN PATENT SPECIFICATION

(11) **EP 3 908 366 B1**
(45) Date of publication and mention of the grant of the patent: **29.01.2025**
(21) Application number: 19816764.5
(22) Date of filing: 12.12.2019
(51) Int. Cl.: A61N 1/362, A61N 1/365, A61B 5/00, A61N 1/37, A61N 1/372, A61B 5/283, A61B 5/349, A61B 5/35

(54) **IMPLANTABLE ASSEMBLY FOR STIMULATING A HUMAN OR ANIMAL HEART**
IMPLANTIERBARE ANORDNUNG ZUR STIMULATION EINES MENSCHLICHEN ODER TIERISCHEN HERZENS
DISPOSITIF IMPLANTABLE POUR STIMULER UN COEUR HUMAIN OU ANIMAL

(30) Priority: 11.01.2019 DE 102019100610; 02.05.2019 EP 19172209
(43) Date of publication of application: 17.11.2021
(73) Proprietor: BIOTRONIK SE & Co. KG, 12359 Berlin (DE)
(72) Inventor: DOERR, Thomas, 12437 Berlin (DE)
(74) Representative: Biotronik Corporate Services SE
(86) International application number: PCT/EP2019/084871
(87) International publication number: WO 2020/143992

(56) References cited:
- US-A1- 2002 120 318
- US-A1- 2012 239 106
- US-A1- 2014 172 035
- US-B2- 8 688 234
- FRÃ HLIG G ET AL: "HIS-BÃ1?4ndel-Stimulation und alternative rechtsventrikulÃ re Stimulationsorte", HERZSCHRITTMACHERTHERAPIE UND ELEKTROPHYSIOLOGIE ; GERMAN JOURNAL OF CARDIAC PACING AND ELECTROPHYSIOLOGY, STEINKOPFF-VERLAG, DA, vol. 19, no. 1, 1 March 2008 (2008-03-01), pages 30 - 40, XP019589206, ISSN: 1435-1544

## Description

The present invention relates to an implantable system for stimulating a human or an animal heart according to claim 1 and to a computer program product according to claim 10.

Implantable systems for stimulating a human or an animal heart, such as cardiac pacemakers, have been known for quite some time. These can carry out a variety of functions. Different stimulation programs may be carried out by a corresponding cardiac pacemaker in the process so as to return the treated heart to a normal state.

Different forms of tachycardias are known. AV nodal reentry tachycardia is the most common type of paroxysmal supraventricular tachycardias among adults. So far, AV nodal reentry tachycardia (AVNRT) has been treated by way of long-term drug therapy using beta blockers or calcium channel blockers. This drug therapy, however, has a high relapse rate. Better, lasting therapy success can be achieved with an ablation therapy, in which damaged myocardial tissue is removed by way of catheter ablation. Conducting an ablation therapy, however, carries the risk of permanently damaging the stimulus conduction system of the heart, which may create AV block. Such a case results in reintervention and entails accordingly increased treatment costs and additional risks for the patient.

US patent 8,688,234 B2 describes a cardiac pacemaker in which antitachycardia pacing of the His bundle can take place. In particular, an XSTIM waveform of the applied stimulation pulse is provided for. Such an XSTIM waveform allows a relatively large virtual electrode to be created, which makes it possible for the electric pulse to penetrate deeply into the cardiac muscle tissue.

It is the object of the present invention to provide an implantable system and a computer program enabling an alternative AV nodal reentry tachycardia therapy option, which significantly reduces or completely eliminates the drawbacks and side effects of the drug therapy or ablation therapy known from the prior art.

This object is achieved by an implantable system for stimulating a human heart or an animal heart having the features of claim 1. Such a system comprises a processor, a memory unit, a stimulation unit, and a detection unit. The stimulation unit is used to stimulate the His bundle of a human heart or an animal heart. It can be suitable, or be specifically provided and configured, for such a stimulation. The detection unit is used to detect an electrical signal of the same heart.

According to the invention, this system is characterized in that the memory unit includes a computer-readable program, which prompts the processor to carry out the steps described hereafter when the program is being executed on the processor.

First, it is ascertained by way of the detection unit whether tachycardia is present in a human heart or an animal heart into which the system is implanted.

If such tachycardia has been identified, a His bundle stimulation is carried out by way of the stimulation unit. The His bundle stimulation is carried out by delivering at least one stimulation pulse. This stimulation pulse has an amplitude in a range of 7.5 V to 30 V, in particular of 12 V to 30 V, in particular of 15 V to 25 V, in particular of 20 V to 22 V, in particular of more than 20 V to 30 V, in particular of more than 20 V to 25 V, and in particular of more than 20 V to 22 V. The stimulation pulse further has a pulse width that is in a range of 1 ms to 15 ms, in particular of 2 ms to 12 ms, in particular of 3 ms to 10 ms, in particular of 4 ms to 9 ms, and in particular of 5 ms to 8 ms.

Particularly effective stimulation of the His bundle can be achieved by a stimulation pulse having such an amplitude and such a pulse width. It is not absolutely necessary for a stimulation electrode of the stimulation unit to be arranged directly in the His bundle in the process. Rather, it is also possible to achieve a stimulation of the His bundle by a stimulation pulse having such a stimulation amplitude and such a pulse width if the corresponding stimulation electrode is arranged on the His bundle or in the vicinity of the His bundle. This simplifies the implantation of such a stimulation electrode. The reason is that the exact capture of the His bundle for a subsequent effective stimulation of the His bundle is not as crucial in the case of such a specification of the stimulation pulse as in other cases. However, even with the selected stimulation amplitude and the selected pulse width of the stimulation pulse, exact capture of the His bundle facilitates a subsequent stimulation of the His bundle.

In one variant, the program prompts the processor to classify a detected tachycardia into one of at least two classes. In this variant, the implantable system is consequently able to discriminate between different tachyarrhythmias of the heart. Tachyarrhythmias of the heart in which a stimulation of the His bundle promises to be a particularly good treatment can then be distinguished from other tachyarrhythmias in which a treatment of the His bundle is less promising to produce success. In one variant, triggering a stimulation of the His bundle can depend on the class into which the detected tachycardia is classified.

In one variant, a first of the at least two classes is provided for AV nodal reentry tachycardia (AVNRT). The reason is that a stimulation of the His bundle is particularly suitable in the case of an AV nodal reentry tachycardia for ending the AV nodal reentry tachycardia, and to restore the treated heart to a normal state. It is not necessary to additionally administer medication or carry out ablation therapy. Rather, solely stimulating the His bundle is sufficient to end a detected AV nodal reentry tachycardia. The device claimed according to the invention is thus, in particular, an obvious choice for treating a detected AV nodal reentry tachycardia, wherein this treatment does not have the serious side effects and risks of the treatment methods of an AV nodal reentry tachycardia known from the prior art.

In one variant, the program prompts the processor to carry out the His bundle stimulation only if the tachycardia was identified as an AV nodal reentry tachycardia. In this variant, the implantable system is only used to treat the AV nodal reentry tachycardia, but not to treat other tachyarrhythmias of the heart and not to treat other tachyarrhythmias of the heart by way of His bundle stimulation.

In one variant, the program prompts the processor to evaluate the signals of an electrocardiogram so as to ascertain whether tachycardia is present. This evaluation can take place in the detection unit or in a separate evaluation unit, for example. A morphological analysis of the electrocardiogram can take place in the process, for example. A morphological analysis criterion of the corresponding electrocardiogram is suitable for this purpose. So as to identify this morphological analysis criterion, a pattern recognition or image recognition step can be carried out in the electrocardiogram, for example, so as to identify certain patterns typical of the presence of tachycardia.

In one variant, the electrocardiogram is an intracardiac electrogram or a far field electrocardiogram (or far field ECG). It is also possible to evaluate both an intracardiac electrogram and a far field ECG.

In one variant, the program prompts the processor to evaluate a chronological sequence of atrial and ventricular signals in the electrocardiogram so as to ascertain whether tachycardia is present. Substantially simultaneously occurring atrial and ventricular signals, at an increased heart rate, are indicative of an AV nodal reentry tachycardia. In such a case, the detected tachycardia can then be classified as an AV nodal reentry tachycardia.

In one variant, the His bundle stimulation is coupled by the stimulation unit to an intrinsic tachycardic excitation of the heart to be treated using a defined or predefinable coupling interval. The program includes appropriate commands, which prompt the processor to accordingly activate the stimulation unit.

In one variant, the program prompts the processor to carry out the His bundle stimulation in the form of a pulse sequence comprising at least two pulses. In one variant, a time delay that is smaller than the cycle length of the ascertained tachycardia is selected between two pulses of this pulse sequence. This means that, in this variant, the His bundle stimulation takes place in the form of what is known as overdrive stimulation.

In another variant, a pulse sequence comprising at least two pulses is also used for His bundle stimulation. However, in this variant, a time delay that is greater than the cycle length of the ascertained tachycardia is selected between two pulses of the pulse sequence. This means that, in this variant, the His bundle stimulation takes place in the form of what is known as underdrive stimulation.

The decision as to whether an overdrive stimulation or an underdrive stimulation is carried out can be made, for example, based on the specific characteristics of the ascertained tachycardia, and in particular of the AV nodal reentry tachycardia.

In one variant, the program prompts the processor to adapt a time delay between two pulses of a pulse sequence comprising at least two pulses which is used for His bundle stimulation, to a previously ascertained cycle length of the detected tachycardia, and in particular of the detected AV nodal reentry tachycardia, using a freely selectable adaptation factor. The His bundle stimulation then takes place with exactly the same characteristics, in terms of time, as the ascertained tachycardia, the His bundle stimulation thus being synchronized with the detected tachycardia.

In another variant, the program prompts the processor to carry out the His bundle stimulation using a single pulse. When such a single pulse is used, a His bundle stimulation can be carried out particularly easily since a chronological sequence of two or more pulses does not need to be taken into consideration. In one variant, however, a single stimulation pulse is coupled to the intrinsic excitation of the heart to be treated, that is, to the intrinsic excitation of the heart.

One aspect of the present invention relates to a computer program product including computer-readable code, which prompts a processor to carry out the steps described hereafter when the code is being executed on the processor.

First, it is detected by way of a detection unit whether tachycardia is present in a human heart or an animal heart.

If such tachycardia has been identified, a His bundle stimulation is carried out by way of a stimulation unit. At least one stimulation pulse is delivered by the stimulation unit in the process. This stimulation pulse has an amplitude in a range of 7.5 V to 30 V. The stimulation pulse further has a pulse width that is in the range of 1 ms to 15 ms.

An illustrative example which, however, is not part of the present invention relates to a method for treating a human patient or an animal patient in need of such treatment. This treatment is carried out by way of an implantable system for stimulating the heart of the patient. This system is implanted in the patient in the process. The system comprises a processor, a memory unit, a stimulation unit, and a detection unit. The stimulation unit is used to stimulate the His bundle of the heart of the patient. It can be suitable, or be specifically provided and configured, for such a stimulation. The detection unit is used to detect an electrical signal of the heart of the patient. The method comprises the steps described hereafter.

First, it is detected by way of the detection unit whether tachycardia is present in the heart of the patient.

If such tachycardia has been detected, a His bundle stimulation is carried out by way of a stimulation unit. In the process, at least one stimulation pulse is delivered by the stimulation unit for the His bundle stimulation. This stimulation pulse has an amplitude in a range of 7.5 V to 30 V. Moreover, the stimulation pulse has a pulse width that is in a range of 1 ms to 15 ms.

One aspect of the present invention relates to an implantable system for stimulating a human heart or an animal heart, having the features described hereafter. Such a system comprises a first stimulation unit and a first detection unit. The first stimulation unit is used to stimulate at least one ventricle of a human heart or of an animal heart. The first detection unit is used to detect an electrical signal of at least one ventricle of the same human or animal heart. This may, for example, be an electrical signal that was generated as a result of a prior stimulation by the stimulation unit. However, this may also be an electrical signal of the ventricle that was generated intrinsically (without prior external stimulation) by the ventricle.

According to the invention, it is provided that the implantable system comprises a second stimulation unit, which is specifically designed and configured to stimulate the His bundle of the same human or animal heart. This means that a stimulation of the His bundle of the heart does not take place by way of the conventionally used stimulation unit, but by way of a separate stimulation unit, which is likewise provided in the system. This second stimulation unit is optimized with respect to the requirements that exist on the part of the His bundle in terms of pacing. In detail, the second stimulation unit is thus specifically designed for His bundle stimulation and configured to have a maximum stimulation energy that is at least 10% higher than the maximum stimulation energy of the first stimulation unit. As a result of this higher maximum stimulation energy, better stimulation of the His bundle can take place. The reason is that, compared to other regions of the heart, the His bundle is comparatively difficult to excite, thereby typically requiring more energy. In contrast, such a higher stimulation energy is not necessary or provided with conventional cardiac pacemakers. These are, in general, only used to stimulate those cardiac regions that can already be easily stimulated using a lower stimulation energy.

In one variant, the stimulation energy of the second stimulation unit is at least 15% higher, in particular at least 20% higher, in particular at least 25% higher, in particular at least 30% higher, in particular at least 35% higher, in particular at least 40% higher, in particular at least 45% higher, in particular at least 50% higher, in particular at least 60% higher, in particular at least 70% higher, in particular at least 80% higher, in particular at least 90% higher, and in particular at least 100% higher than the maximum stimulation energy of the first stimulation unit.

In one variant, the maximum stimulation energy of the second stimulation unit is higher than the maximum stimulation energy of the first stimulation unit by a defined percentage, which is formed for an interval of the aforementioned percentages. This means that, in this variant, the maximum stimulation energy of the second stimulation unit is, in particular, higher than the maximum stimulation energy of the first stimulation unit by a percentage that ranges between 10% and 100%, in particular between 15% and 90%, in particular between 20% and 80%, and so forth.

So as to be able to detect an excitation of the His bundle or an activity of the His bundle particularly easily and reliably, the system, in one variant, comprises a second detection unit, which is specifically designed and configured to detect an electrical signal of the His bundle of the same human or animal heart. The specific design and configuration of the second detection unit for detecting electrical signals of the His bundle may be achieved in a variety of ways. For example, the sensitivity of the second detection unit can be at least 10% higher than the sensitivity of the first detection unit. As an alternative or in addition, the detection range of the second detection unit can be at least 10% greater than the detection range of the first detection device. As an alternative or in addition, the sampling rate of the second detection unit can be at least 10% higher than the sampling rate of the first detection unit. Such enhanced sensitivity and/or such a greater detection range and/or such a higher sampling rate compared to a conventional detection unit makes it possible to detect signals of the His bundle particularly easily, or in the first place.

In one variant, the sensitivity of the second detection unit is at least 15% higher, in particular at least 20% higher, in particular at least 25% higher, in particular at least 30% higher, in particular at least 35% higher, in particular at least 40% higher, in particular at least 45% higher, in particular at least 50% higher, in particular at least 60% higher, in particular at least 70% higher, in particular at least 80% higher, in particular at least 90% higher, and in particular at least 100% higher than the sensitivity of the first detection unit.

In one variant, the sensitivity of the second detection unit is higher than the sensitivity of the first detection unit by a defined percentage, which is formed of an interval of the aforementioned percentages. This means that, in this variant, the sensitivity of the second detection unit is, in particular, higher than the sensitivity of the first detection unit by a percentage that ranges between 10% and 100%, in particular between 15% and 90%, in particular between 20% and 80%, and so forth.

In one variant, the detection range of the second detection unit is at least 15% greater, in particular at least 20% greater, in particular at least 25% greater, in particular at least 30% greater, in particular at least 35% higher, in particular at least 40% greater, in particular at least 45% greater, in particular at least 50% greater, in particular at least 60% greater, in particular at least 70% greater, in particular at least 80% greater, in particular at least 90% greater, and in particular at least 100% greater than the detection range of the first detection unit.

In one variant, the detection range of the second detection unit is greater than the detection range of the first detection unit by a defined percentage, which is formed of an interval of the aforementioned percentages. This means that, in this variant, the detection range of the second detection unit is, in particular, greater than the detection range of the first detection unit by a percentage that ranges between 10% and 100%, in particular between 15% and 90%, in particular between 20% and 80%, and so forth.

In one variant, the sampling rate of the second detection unit is at least 15% higher, in particular at least 20% higher, in particular at least 25% higher, in particular at least 30% higher, in particular at least 35% higher, in particular at least 40% higher, in particular at least 45% higher, in particular at least 50% higher, in particular at least 60% higher, in particular at least 70% higher, in particular at least 80% higher, in particular at least 90% higher, and in particular at least 100% higher than the sampling rate of the first detection unit.

In one variant, the sampling rate of the second detection unit is higher than the sampling rate of the first detection unit by a defined percentage, which is formed of an interval of the aforementioned percentages. This means that, in this variant, the sampling rate of the second detection unit is, in particular, higher than the sampling rate of the first detection unit by a percentage that ranges between 10% and 100%, in particular between 15% and 90%, in particular between 20% and 80%, and so forth.

In one variant, the system comprises a first timer, which is used to deliver stimulation pulses by the first stimulation unit in a chronologically defined manner. In addition, the system, in this variant, comprises a second timer, which is provided and configured to match the delivery point in time at which at least one pulse to be delivered by the second stimulation unit to a delivery point in time of at least one pulse to be delivered by the first stimulation unit. Whereas the implantable systems for stimulating the human or animal heart known from the prior art frequently comprise a single timer, which can be used to carry out a stimulation therapy in a chronological sequence that is meaningful for the heart to be stimulated, the provision of a second timer, which ensures synchronization between the second stimulation unit and the first stimulation unit, is not known from the prior art. This second timer thus allows the stimulation of the His bundle to be synchronized with the stimulation of another cardiac region by the first stimulation unit. In this way, a synergistic effect can be achieved particularly easily between the His bundle stimulation and conventional stimulation of another cardiac region.

In one variant, the second timer is not only used to synchronize a His bundle stimulation with a conventional stimulation of another cardiac region, but also to synchronize such a conventional stimulation of a cardiac region with a His bundle stimulation to be carried out by the same implantable system. It is provided in this variant, for example, that the second timer is also used to match the delivery point in time of at least one pulse that is to be delivered by the first stimulation unit to a point in time at which at least one pulse is to be delivered by the second stimulation unit.

In one variant, the system comprises a first stimulation threshold test device. Such a stimulation threshold test device can be used to ascertain a stimulus threshold of the His bundle of a human heart or an animal heart which is to be stimulated by the device. Typically, such a stimulus threshold initially rises after the implantation of a corresponding system, so as to drop to a lower level again in the weeks thereafter. A stimulation threshold test device can be used to ascertain the stimulus threshold that has to be exceeded in the present situation to achieve sufficient stimulation of the His bundle. A stimulation energy of the second stimulation unit can then be adjusted as a function of the ascertained stimulus threshold. When this stimulus threshold is lower than shortly after the implantation, a lower stimulation energy is needed. As a result, the energy expenditure for each stimulation process is considerably reduced, while achieving an equally good stimulation outcome. In this way, the service life of the implantable system can be extended.

The stimulation threshold test device may, for example, carry out automated threshold monitoring (ATM), active tracking of the stimulation energy with every heartbeat (automated capture control, ACC), or automatic tracking of the stimulation energy once or twice a day after a stimulus threshold measurement (automated threshold test, ATT), so as to ascertain the stimulus threshold of the His bundle.

In one variant, the system comprises not only a first stimulation threshold test device, but also a second stimulation threshold test device. Such a second stimulation threshold test device can be used to detect a stimulus threshold of another cardiac region (that is, not of the His bundle) which is to be excited by the first stimulation unit. It is then also possible to ascertain the current stimulus threshold for these cardiac regions, so as to adjust the stimulation energy of the first stimulation unit as a function of this ascertained stimulus threshold. In this way, it is possible to adapt not only the energy required for the His bundle stimulation, but also the energy needed for a conventional stimulation of a cardiac region, to the respectively necessary level. This ensures sufficiently high stimulation energy, which is needed for pacing to be successful. At the same time, the amount of energy expended does not exceed the level needed for pacing, which, as described above, extends the service life of the implantable system. The second stimulation threshold test device can also carry out different processes, such as ATM, ACC and ATT, for ascertaining the stimulus threshold of the particular cardiac regions.

In one variant, the system comprises a memory unit. This memory unit comprises a memory area that is used exclusively for storing data collected by the second detection unit and/or related to an activity of the second stimulation unit. The activity of the second stimulation unit is, in particular, expressed by the type, frequency and number of paces delivered by the second stimulation unit to stimulate the His bundle. This memory area is thus used to record all processes that are detected or carried out by the implantable system and that relate to a stimulation of the His bundle. This allows the implantable system to read out and evaluate a His bundle stimulation separately from other activities of the implantable system.

So as to open up a particularly simple option of drawing on data stored within the implantable system, the system, in one variant, comprises a remote communication unit. This remote communication unit makes it possible to transmit data collected by the second detection unit and/or data related to an activity of the second stimulation unit. As an alternative or in addition, this remote communication unit makes it possible to monitor the second detection unit and/or the second stimulation unit. As an alternative or in addition, the remote communication unit further makes it possible to adapt a stimulation to be delivered by the second stimulation unit. This means that remote access to the second stimulation unit is possible via the remote communication unit. In this way, it is possible to remotely adapt stimulation parameters of a His bundle stimulation to be carried out. Likewise, data reflecting a prior His bundle stimulation can be remotely retrieved from the implantable system by the remote communication unit. Finally, the remote communication unit allows correct functioning of the second detection unit and/or of the second stimulation unit to be checked remotely.

In one variant, the implantable system comprises a processor and a memory unit. The memory unit may be the memory unit already described above, which includes a specific memory area that is reserved for events related to the activity or stimulation of the His bundle. According to the above-described variant, this memory unit includes a computer-readable program, which prompts the processor to carry out the steps described hereafter when the program is being executed on the processor. First, the system is transferred into a His bundle stimulation mode, in which only His bundle stimulation can be carried out by way of the second stimulation unit. This means that the first stimulation unit, which can enable a conventional stimulation of another cardiac region, is deactivated in this His bundle stimulation mode. Thereafter, a His bundle stimulation in the form of electrical pulses is delivered by way of the second stimulation unit, which is to result in a stimulation of the His bundle. This delivery takes place when an event warranting the delivery was detected by way of the first detection unit and/or by way of the second detection unit. Thus, if, for example, skipping of the natural heartbeat or a non-physiological decrease in the heart rate was identified, the His bundle can be stimulated by way of the second stimulation unit so as to restore a normal heart rhythm. If, thereafter, the first detection unit and/or the second detection unit detect a normal heart rhythm again, no further stimulation by the implantable system, and in particular, no conventional stimulation of another region of the heart by the first stimulation unit, is required.

In another variant, the system also comprises a processor and a memory unit. This may again be the same memory unit that includes a memory area reserved for the His bundle stimulation, and that optionally includes a program by which the system can be transferred into a His bundle stimulation mode. However, it is also possible for the memory unit discussed hereafter to be a memory unit that is different from the previous memory unit. In this variant, the memory unit includes a computer-readable program, which prompts the processor to carry out the steps described hereafter when the program is being executed on the processor. First, the system is transferred into a safety mode. In this safety mode, it is possible both to stimulate at least one ventricle by way of the first stimulation unit, and to carry out a His bundle stimulation by way of the second stimulation unit. This means that the safety mode is used to enable a fundamental activatability of the first stimulation unit and of the second stimulation unit. Thereafter, a ventricular stimulation is delivered by way of the first stimulation unit and/or a His bundle stimulation is delivered by way of the second stimulation unit. This delivery takes place when an event warranting the delivery was detected by way of the first detection unit and/or by way of the second detection unit. The safety mode thus focuses not only on a His bundle stimulation, but is also used to carry out a conventional stimulation of any cardiac region on which the first stimulation unit can act, simultaneously with or with delay from the His bundle stimulation.

In one variant, the system comprises a marker channel. Such a marker channel can be used to read out an electrocardiogram (ECG) or an intracardiac electrogram (IEGM). The electrocardiogram or the intracardiac electrogram has at least one marking that is specific to a His bundle stimulation by way of the second stimulation unit and/or specific to a detection of a signal of the His bundle by way of the second detection unit. A specific marking of the electrocardiogram that can be read out or of the intracardiac electrogram that can be read out with a His bundle-specific marking thus takes place. In other words, His bundle-specific markers are used to provide the ECG or IEGM with such His bundle-specific markings. A read-out of the ECG or of the IEGM can take place by way of real time telemetry. As an alternative, it is also possible for the ECG and/or the IEGM to be stored in the system or in a memory unit of the system, and to be read out subsequently at a later point in time. A remote transmission of ECGs or IEGMs is possible in the process. In particular, a remote transmission unit can be used for this purpose. For example, the remote transmission unit described above is suitable for this purpose.

So as to render a use of a system according to the invention particularly easy for a user and avoid incorrect use, the system, in one variant, comprises a first stimulation output and a second stimulation output. The first stimulation output is used to connect a stimulation electrode, which forms part of the first stimulation unit. The second stimulation output is used to connect a second stimulation electrode, which forms part of the second stimulation unit. The first stimulation electrode can be used to stimulate any cardiac region in one of the two atria or ventricles (not, however, the His bundle). The second stimulation electrode, in contrast, is used to specifically stimulate the His bundle of the human or animal heart to be treated. So as to enable a connection of the first stimulation electrode to the first stimulation output and, in particular, a correct connection of the second stimulation electrode to a second stimulation output, at least the second stimulation output is provided with a special marking, which clarifies to a user that the second stimulation output is a stimulation output that is provided and configured for connecting a His bundle stimulation electrode. This special marking may, for example, be a text marking and/or a color marking of the stimulation output. The marking may be provided directly on the stimulation output or in a region of the implantable system that surrounds the stimulation output or adjoins the stimulation output. For example, a label or a sticker can be provided on the implantable system, which identifies the corresponding specific design of the second stimulation output as a His bundle stimulation output.

As an alternative or in addition, it may further be provided to provide an appropriate marking on the packaging of the implantable system. As an alternative or in addition, an appropriate marking is provided in an accompanying document of the implantable system (such as operating instructions or a user manual). As an alternative or in addition, such a marking is provided on an electrode lead of the second stimulation electrode. For example, the electrode lead of the second stimulation electrode can include a specific color code that matches a color code of the second stimulation output. This is a particularly simple way to visualize for a user which stimulation electrode is to be plugged into which stimulation output of the implantable system.

As an alternative or in addition, a marking indicating that the second stimulation output is configured as a His bundle stimulation output may also be provided on a programming device, which is used to program the implantable system. As an alternative or in addition, such a marking may also be provided in a remote monitoring system. For example, an appropriate marking may be present on a graphical user interface (GUI) of the remote monitoring system, visualizing to a user which of the stimulation outputs present is a conventional stimulation output, and which output is a His bundle stimulation output. Typically, it suffices to mark the His bundle stimulation output, since unmarked stimulation outputs are typically considered to be conventional stimulation outputs by a user.

One aspect of the present invention relates to a computer program product including computer-readable code, which prompts a processor to carry out the steps described hereafter when the code is being executed on the processor.

First, it is detected by way of a first detection unit and/or a second detection unit whether a cardiac rhythm disturbance to be treated is present in a human heart or an animal heart. The first detection unit is provided to detect an electrical signal of at least one ventricle of the human or animal heart. The second detection unit is specifically designed and configured to detect an electrical signal of the His bundle of the same human or animal heart.

If a cardiac rhythm disturbance to be treated is present, atrial or ventricular stimulation is subsequently carried out by way of a first stimulation unit and/or a His bundle stimulation is carried out by way of a second stimulation unit.

Another illustrative example which, however, is not part of the present invention relates to a method for treating a human patient or an animal patient in need of such treatment. This method is carried out using an implantable system according to the above descriptions. Such a system comprises a first stimulation unit and a first detection unit. The first stimulation unit is used to stimulate at least one ventricle of the heart of the patient. The first detection unit is used to detect an electrical signal of at least one ventricle of the heart of the patient.

The method is characterized in that the system comprises a second stimulation unit, which is specifically designed and configured to carry out a stimulation of a His bundle of the heart of the patient. For the purpose of this specific design and configuration, the second stimulation unit has a maximum stimulation energy that is at least 10% higher than the maximum stimulation energy of the first stimulation unit. Optionally, a second detection unit can be present, which is specifically designed and configured to detect an electrical signal of the His bundle of the heart of the patient.

According to the method, it is provided that, when an event warranting treatment is detected in the heart of the patient by way of the first detection unit and/or the second detection unit, a ventricle of the patient is stimulated by way of the first stimulation unit and/or the His bundle of the patient is stimulated by way of the second stimulation unit. A stimulation of the His bundle of the heart of the patient results in a simultaneous stimulation of both ventricles. In contrast, only one ventricle of the patient can typically be directly excited by the first stimulation unit. A stimulation of the His bundle is thus suitable, in particular, for biventricular stimulation and for ventricular resynchronization.

One aspect of the present invention relates to an implantable system for stimulating a human heart or an animal heart, having the features described hereafter. Such a system comprises a processor, a memory unit, a stimulation unit, and a detection unit. The stimulation unit is used to stimulate the His bundle of a human heart or an animal heart. The detection unit is used to detect an electrical signal of the same heart.

According to the invention, the system is characterized in that the memory unit includes a computer-readable program, which prompts the processor to carry out the steps described hereafter when the program is being executed on the processor.

First, a cardiac stimulation is carried out by way of the stimulation unit.

Thereafter, a cardiac electrical signal is detected by way of the detection unit. This cardiac electrical signal was generated by cardiac excitation caused by the cardiac stimulation carried out beforehand.

The detected electrical signal is then used to ascertain the excitation state of the heart stimulated by the cardiac stimulation.

When this excitation state has been ascertained, it is classified into one of at least three classes. These at least three classes include a first class that is provided for a first excitation state, and a second class that is provided for a second excitation state. The second excitation state differs from the first excitation state. A third class of the at least three classes is provided for an unsuccessful stimulation without a detectable excitation state. Using this classification, it is thus possible to distinguish two different cardiac excitation states from one another and, additionally, to distinguish these together from an unsuccessful stimulation. Only an extremely weak cardiac electrical signal, or a cardiac electrical signal that does not set itself apart or that sets itself apart only slightly from the background, is detected in the case of such an unsuccessful stimulation. Such a signal indicates that the stimulation carried out beforehand has not resulted in cardiac excitation with a pronounced cardiac electrical response signal, that is, it was unsuccessful.

Afterwards, at least one control parameter of the system is automatically adapted as a function of the classification that was carried out. This means that the assignment of the ascertained excitation state to one of the at least three classes is the cause as to how the adaptation of the at least one control parameter is carried out. The achieved stimulation outcome is thus directly taken into consideration by the implantable system, so that subsequent stimulations are optimized with respect to the physiological needs of the heart to be stimulated.

To the extent that the ascertained excitation state shows that this state corresponds exactly to the expected value, a separate adaptation of the control parameter is not required. Rather, it would then be actively decided that no automatic adaptation has to be carried out.

The automatically adaptable control parameter is selected from a His bundle stimulation energy, a His bundle stimulation vector, an operating mode of the system, at least one parameter of a timer of the system, and a stimulation control parameter suitable for cardiac resynchronization therapy.

An operating mode of the system may, for example, be a His bundle stimulation operating mode and a conventional stimulation operating mode in which the algorithms used are not adapted to a stimulation of the His bundle. Further suitable operating modes are a right ventricular backup stimulation and a left ventricular backup stimulation. The parameters of a timer of the system are used to coordinate the chronological sequence of different stimulations or of individual pulses within a stimulation in terms of time. For example, such timers are typically responsible for defining a time interval between a first stimulation and a second stimulation.

The automatic adaptation, as needed, of the control parameter of the system as a function of the classification of the excitation state of the stimulated heart carried out beforehand allows the operation of the implantable system to be optimized with respect to the His bundle stimulation.

In one variant, the stimulation unit is not only suitable for stimulating the His bundle of a human or an animal heart, but is specifically designed and configured to do so. This can, in particular, be achieved via the maximum stimulation energy delivered by the stimulation unit, or via the pulse width of the pulses delivered by the stimulation unit.

In one variant, suitable stimulation amplitudes of the pulses delivered by the stimulation unit are in a range of 12 V to 30 V, in particular 15 V to 25 V, and in particular 20 V to 22 V.

Suitable pulse widths of the pulses delivered by the stimulation unit are in a range between 1 ms and 15 ms, in particular between 2 ms and 12 ms, in particular between 3 ms and 10 ms, in particular between 4 ms and 9 ms, and in particular between 5 ms and 8 ms.

Likewise, the detection unit can either be particularly suitable for detecting His bundle-specific excitations pulses of the heart, or be specifically designed and configured to do so. For example, the detection unit can comprise a low-pass filter, which is particularly suitable for signals generated by an excitation of the His bundle. In one variant, such a low-pass filter has a cut-off frequency of at least 1 kHz (that is, 1 kHz or greater), in particular at least 500 Hz, in particular at least 200 Hz, and most particularly at least 100 Hz. In one variant, such a low-pass filter has a cut-off frequency in a range between 100 Hz and 1 kHz, in particular between 200 Hz and 500 Hz, and in particular between 100 Hz and 200 Hz. Such a low-pass filter essentially allows signal components having a frequency below the cut-off frequency to pass unimpaired. In contrast, signal components having a frequency greater than the cut-off frequency are weakened or attenuated.

The particular suitability of the detection unit for detecting His bundle-specific pulses, or the design and configuration of the detection unit for this purpose, can also be implemented by a sampling rate that, in one variant, is at least 500 Hz, in particular at least 1 kHz, in particular at least 2 kHz, in particular at least 5 kHz, and most particularly at least 10 kHz. In one variant, the sampling rate of the detection unit is in a frequency range between 500 Hz and 10 kHz, in particular between 1 kHz and 5 kHz, and in particular between 2 kHz and 4 kHz.

In one variant, the detection unit has a sensitivity in a range between 0.05 mV and 0.25 mV, and in particular between 0.1 mV and 0.2 mV. This kind of sensitivity of the detection unit is particularly well-suited for being able to detect His bundle-specific electrical cardiac signals.

As was already mentioned, one of the at least three classes is a class that is provided for an unsuccessful stimulation without a detectable excitation state. In one variant, the remaining classes of the at least three classes (that is, the first class, the second class and an optionally provided further class) are selected from the classes described hereafter. One class relates to an excitation state of the heart for which only a stimulation of the His bundle of the stimulated heart has taken place. This means that an excitation state in which a selective stimulation of the His bundle has taken place falls into this class. Another class relates to an excitation state for which both a stimulation of the His bundle of the heart and a stimulation of the ventricular myocardium have taken place. This class thus relates to an excitation state that is based on non-selective stimulation. One class relates to an excitation state for which only a stimulation of the ventricular myocardium of the stimulated heart has taken place. This means that this excitation state is based on a stimulation in which the His bundle was not captured. This may be referred to as a purely ventricular excitation state. One class relates to an excitation state in which a His bundle stimulation and a stimulation of left ventricular conduction pathways have taken place. Another class relates to an excitation state in which a His bundle stimulation without stimulation of left ventricular conduction pathways has taken place. Such a stimulation is also referred to as a right bundle branch block (RBBB).

The classification of the excitation state into one of the predefined classes automatically implies a classification of the stimulation underlying the excitation state into such a class.

In one variant, the program prompts the processor to carry out an automatic stimulation threshold test (automated threshold monitoring, ATM) once or several times a day. Within the scope of such a stimulation threshold test, a stimulus threshold for a successful His bundle stimulation is determined. A selective or non-selective His bundle stimulation is considered successful, whereas a stimulation in which the His bundle is not captured or which only results in ventricular stimulation is not considered successful.

In one variant, the program prompts the processor to classify each cardiac stimulation carried out by way of the stimulation unit. For this purpose, for example, active capture control (ACC) can be employed, optionally using a beat-to-beat algorithm.

In one variant, the system is designed as a single channel stimulator, which has only one terminal for a stimulation and detection electrode. The stimulation and detection electrode forms part of the stimulation unit and of the detection unit in the process. As a result, it may also be referred to as a His bundle stimulation and detection electrode or as a His bundle stimulation and detection channel. The detection unit is not only provided and configured for detecting His bundle-specific cardiac signals of the heart to be stimulated. Rather, it is also provided and configured for detecting an intrinsic excitation of one of the two atria of the heart to be stimulated. Signals resulting from such an intrinsic excitation of one of the two atria are also referred to as a P wave. When the detection unit is provided and configured for detecting such atrial signals, the stimulation of the His bundle of the heart to be stimulated can be triggered or carried out particularly easily by the stimulation unit as a function of these intrinsic atrial signals.

In one variant, the implantable system is designed as a two-channel stimulator. The system then comprises a first terminal for a His bundle stimulation and detection electrode (or for a His bundle stimulation and detection channel), and a second terminal, which can be designed as an atrial stimulation and detection channel, or as a ventricular stimulation and detection channel, for connecting a corresponding atrial stimulation and detection electrode or a ventricular stimulation and detection electrode. In such a variant, the system typically comprises a second stimulation unit and a second detection unit, to which the atrial or ventricular stimulation and detection electrode is then assigned, in addition to the stimulation unit and the detection unit.

In one variant, the implantable system is designed as a three-channel stimulator. Such a three-channel stimulator comprises a first terminal for a His bundle stimulation and detection channel (or for a His bundle stimulation and detection electrode), a second terminal for an atrial stimulation and detection channel (or for an atrial stimulation and detection electrode), and a third terminal for a ventricular stimulation and detection channel (or for a ventricular stimulation and detection electrode). Such a three-channel stimulator can be used both to carry out His bundle-specific stimulations and detect His bundle-specific signals, and to carry out atrial and ventricular stimulations and detect atrial and ventricular signals particularly easily.

In one variant, the implantable system is designed as a four-channel stimulator. Such a four-channel stimulator comprises a first terminal for a His bundle stimulation and detection channel (or for a His bundle stimulation and detection electrode), a second terminal for an atrial stimulation and detection channel (or for an atrial stimulation and detection electrode), a third terminal for a first ventricular stimulation and detection channel (or a first ventricular stimulation and detection electrode), and a fourth terminal for a second ventricular stimulation and detection channel (or for a second ventricular stimulation and detection electrode). Such a four-channel stimulator can be used, for example, to stimulate both ventricles of a human heart or an animal heart simultaneously. Similarly, it is possible to detect signals from both ventricles simultaneously. Furthermore, the His bundle-specific stimulation and detection already described in connection with the preceding variants, and atrial stimulation and detection, are possible.

In another variant, the implantable system is designed as a five-channel stimulator, which has a total of five terminals. A first terminal is provided for a His bundle stimulation and detection channel (or for a His bundle stimulation and detection electrode). A second terminal is provided for an atrial stimulation and detection channel (or for an atrial stimulation and detection electrode). A third terminal is used to connect a first ventricular stimulation and detection channel (or a first ventricular stimulation and detection electrode). A fourth terminal is provided for a second ventricular stimulation and detection channel (or for a second ventricular stimulation and detection electrode). Finally, a fifth terminal is provided for a third ventricular stimulation and detection channel (or for a third ventricular stimulation and detection electrode). The implantable system can thus be used as a stimulator, for example, which is configured for cardiac resynchronization (CRT) (CRT stimulator) and has a quadripolar left ventricular channel. As an alternative, the implantable system can enable multipolar stimulation (known as multipole pacing), using a His bundle-specific stimulation channel.

In one variant, the implantable system comprises a device for automatic cardioversion and/or defibrillation of the heart and is configured with appropriate terminals to connect this device to suitable cardiac regions.

In one variant, the system comprises a stimulation outcome monitoring unit, which is configured as a module that, in terms of hardware, is implemented separately from the stimulation unit and the detection unit. This stimulation outcome monitoring unit assumes at least some of the steps by which the outcome of a cardiac stimulation carried out beforehand is monitored, and in particular, the steps for ascertaining the excitation state of the stimulated heart and for classifying the excitation state in one of at least three classes. Such a stimulation outcome monitoring unit can then send suitable control signals to the stimulation unit or a control unit of the implantable system, so as to achieve an automatic adaptation of at least one control parameter of the system.

For the His bundle stimulation, in principle at least two stimulus thresholds are of importance in the stimulation unit. The first of these stimulus thresholds is the stimulus threshold for stimulating the ventricular myocardium. This stimulus threshold is comparable to the tissue stimulus threshold of conventional stimulation channels and depends, in particular, on the electrical connection of the corresponding electrode to the myocardium, and the fundamental stimulation capability properties of the particular myocardium. The second stimulus threshold is the stimulus threshold of the actual His bundle stimulation. It is necessary for this stimulus threshold to be exceeded so as to sufficiently excite the His bundle structures, so that the effects of the implemented His bundle stimulation are achieved, and in particular, so that excitation propagation is achieved.

It is not possible with conventional systems for stimulating a human heart or an animal heart, such as conventional cardiac pacemakers, to monitor the progression of both stimulus thresholds. Rather, it is always only a single stimulus threshold per stimulation channel that is recorded and stored. If, however, only one of the two relevant stimulus thresholds can be recorded and stored, there is a risk of faulty operation and of a misinterpretation of the stored results. There is a significant risk of confusion, in particular, when different persons are entrusted with identifying and storing the corresponding stimulus threshold. If a first person identifies and stores the first stimulus threshold (that is, the stimulus threshold of the ventricular myocardium) as a relevant stimulus threshold, while another person identifies and stores the second stimulus threshold (that is, the His bundle stimulus threshold) as a relevant stimulus threshold, misinterpretations could result during the monitoring of the progression of the stimulus threshold. For example, if the stimulus threshold of the His bundle stimulation is accidentally stored and evaluated, at least intermittently, instead of the stimulus threshold of the ventricular myocardium, it is possible that the misdiagnosis is made that the stimulus threshold has risen in general, which indicates poorer stimulation capability, which could, for example, be caused by necrotic tissue. In such a case, however, the proportion of necrotic tissue in the heart would, in fact, not have changed, and only that the "incorrect" stimulus threshold value was being considered.

In one variant, the program thus prompts the processor to determine at least two stimulus threshold measurement values at the same point in time, and to store these in a measurement value memory. A first stimulus threshold measurement value indicates a stimulus threshold of a first excitation state of the stimulated heart, whereas a second stimulus threshold measurement value indicates a stimulus threshold of a second excitation state of the stimulated heart.

The measurement value memory can be a defined memory area of the memory unit, or may also be implemented as a memory designed separately from the memory unit. The synchronous collection of two different stimulus threshold measurement values opens up the option of monitoring two different stimulus thresholds, and to thus draw different medical conclusions from these monitored stimulus thresholds.

In one variant, the first excitation state and the second excitation state are selected from the excitation states described hereafter. This may be an excitation state for which only a stimulation of the His bundle of the stimulated heart has taken place (selective stimulation). The excitation state can further be a state for which both a stimulation of the His bundle of the stimulated heart and a stimulation of the ventricular myocardium have taken place (non-selective stimulation). Finally, the excitation state can be a state for which only a stimulation of the ventricular myocardium of the stimulated heart (without stimulation of the His bundle) has taken place.

Such a breakdown of the different excitation states, and the assignment of the at least two determined stimulus threshold measurement values to such an excitation state, thus makes it possible to distinguish the stimulus threshold of a His bundle stimulation from the stimulus threshold of a ventricular myocardium stimulation. It is thus possible, for example, to identify a histological change of the stimulated heart based on a rise in the stimulus threshold of a ventricular myocardium stimulation which occurs chronologically over time. The reason is that when the stimulus threshold that is relevant for the ventricular myocardium stimulation increases, this is indicative of an overall poorer stimulation capability of the human or animal to be stimulated, which may be caused by an increase in the proportion of necrotic tissue.

If, in contrast, the stimulus threshold that is relevant for the His bundle stimulation rises chronologically over time, this is not indicative of a poorer stimulation capability of the heart as a whole, but rather of a (possibly only minor) dislocation of the corresponding stimulation electrode. By monitoring two different stimulus thresholds separately, it is thus possible to obtain medically relevant data, which significantly reduces the risk of a misdiagnosis, and thus considerably influences patient safety.

One aspect of the present invention thus relates to an implantable system for stimulating a human heart or an animal heart in which such a collection of at least two stimulus threshold measurement values, including the variants of this stimulus threshold measurement value collection described above and hereafter, is provided, but which does not include a classification of a verified excitation state into one of at least three predefined classes, and does not include a subsequent automatic adaptation of a control parameter of the system as a function of the classification that was carried out. Such a system can also be described as follows:
An implantable system for stimulating a human heart or an animal heart, comprising a processor, a memory unit, a stimulation unit for stimulating a His bundle of a human heart or an animal heart, and a detection unit for detecting an electrical signal of the same heart, characterized in that the memory unit includes a computer-readable program, which prompts the processor to carry out the following steps when the program is being executed on the processor:
a) carrying out a cardiac stimulation by way of the stimulation unit;
b) detecting a cardiac electrical signal, which was generated by a cardiac excitation as a result of the cardiac stimulation carried out beforehand, by way of the detection unit;
c) ascertaining an excitation state of a heart stimulated by the cardiac stimulation by way of the electrical signal; and
d) determining two stimulus threshold measurement values at the same point in time and storing the stimulus threshold measurement values in a measurement value memory, a first stimulus threshold measurement value indicating a stimulus threshold of a first excitation state of the stimulated heart, and a second stimulus threshold measurement value indicating a stimulus threshold of a second excitation state of the stimulated heart.

The variants and exemplary embodiments described above and hereafter refer both to the implantable system for stimulating a human heart or an animal heart in which an excitation state is classified into one of at least three classes and subsequently an automatic adaptation of at least one control parameter is carried, and to those variants in which this classification and automatic adaptation are not carried out. In addition, the variants described hereafter can also be combined with further aspects of the present invention.

In one variant, the program prompts the processor to store a chronological progression of the at least two stimulus threshold measurement values. Storing such a progression makes it possible in a particularly simple manner to monitor the stimulus threshold measurement values over time. In this way, a trend of the stimulus threshold measurement values can be identified particularly easily, from which the corresponding conclusions can then be derived with respect to a potential change in the cardiac stimulation capability or a potential dislocation of a stimulation electrode.

In one variant, the system is provided and configured for establishing a warning threshold value for each of the at least two stimulus threshold measurement values. It is provided that the program prompts the processor to output a warning if at least one of the warning threshold values is being exceeded. In this way, it is possible to inform a user of the implantable system about deviations from the expected behavior of the system at an early stage. It is then possible to take actions early on, so as to halt or reverse a potential change in tissue of the human or animal heart to be stimulated, or so as to carry out a relocation of an electrode.

For example, the warning threshold values can be stored in the implantable system itself, in a programming device used to program the implantable system or to program the components thereof, or in a remote monitoring system, which allows the implantable system to be accessed by way of data remote transmission.

In one variant, the stimulus threshold measurement values stored in the measurement value memory each include at least one value pair, which, on the one hand, pertains to the stimulus threshold in the form of an amplitude (measured in volts or a comparable voltage unit) and, on the other hand, pertains to a pulse width (measured in milliseconds or another unit of time). A stimulus threshold can then typically be indicated as a voltage at a particular pulse width. In another variant, additionally a point in time of the measurement is assigned to each stimulus threshold measurement value.

So as to be able to identify the different stimulus thresholds particularly easily, these are provided, in one variant, with the designations "His bundle-selective," "His bundle non-selective" and "only ventricular" or descriptions comparable in terms of meaning. Illustrations on a graphical user interface or in printouts, for example, can thus be accordingly identified, so as to facilitate the work for the medical staff evaluating the corresponding data.

In one variant, the system comprises a data remote transmission unit, by way of which it is possible to transmit the stored stimulus threshold measurement values to a remote monitoring system, for example. For example, stimulus threshold measurement values of numerous different implantable systems for stimulating the heart can be collected and evaluated in such a remote monitoring system. It is then possible, from such a central remote monitoring system, to monitor progressions of stimulus threshold measurement values in different implantable systems and, if necessary, to inform the user of these systems, or to access these systems for corrective purposes.

For a particularly simple evaluation, the stimulus threshold measurement values, in one variant, are represented together with a difference from a predefinable stimulus threshold value. It is then possible to identify particularly easily whether the presently ascertained stimulus threshold measurement value is higher or lower than the predefined or predefinable stimulus threshold value.

So as to achieve a simple distinction of the ascertained stimulus threshold measurement values from one another and a particularly simple assignment to a stimulus threshold type, it is provided, in one variant, that a user can assign at least one of the following descriptions of the selected cardiac stimulation type to the stimulus thresholds: "low atrial stimulation," "combined atrial stimulation and His bundle stimulation," "His bundle stimulation," "combined atrial stimulation, ventricular septal stimulation and His bundle stimulation," "combined ventricular septal stimulation and His bundle stimulation" and "ventricular stimulation." By identifying such stimulation types with real names, it is particularly easy to assign different stimulus thresholds to the respective stimulation types. In this way, a confusion of different stimulus thresholds can be avoided.

In one variant, the program prompts the processor to carry out a signal quality check (SQC) prior to ascertaining the excitation state. Such a signal quality check makes it possible to establish whether the signals provided for the ascertainment of the excitation state are suitable for the evaluation. This suitability can be derived, for example, from the absolute signal intensity, from a signal-to-noise ratio, a signal offset or a signal morphology criterion, such as a signal rise velocity, or the number of zero baseline crossings within a time window.

If the signal quality check shows that the quality of the signals provided for the ascertainment of the excitation state is not sufficient, the classification used for the stimulation outcome monitoring and the subsequent automatic adaptation of at least one control parameter of the system can be at least partially temporarily deactivated. As an alternative, it is also possible to appropriately identify the resultant classification result, that is, to indicate that this result was obtained based on signals that did not pass the signal quality check that was carried out. The corresponding classification is thus identified as being less reliable than other classifications.

In one variant, the program prompts the processor to automatically adapt a signal processing parameter of the system as a function of the signal quality check that was carried out. This may, for example, be an amplification, an attenuation, a filtering, a differentiation, a smoothing, an averaging, a phase shift or an offset correction of the measured signals, so as to be able to achieve a better evaluation of these signals. In this way, it becomes possible to amplify signals that may not pass, or just barely pass, the signal quality check or to ensure that these stand out against the background, so that the quality is nonetheless sufficient, that is, that the signal quality check is passed, thereby enabling a reliable evaluation of the corresponding signals.

In one variant, the program prompts the processor to use a morphological analysis criterion from a derived electrocardiogram, or a morphological analysis criterion from an electrocardiogram that was obtained by way of an electrode belonging to the stimulation unit, for the classification of the excitation state. In this way, it is possible, for example, to carry out a pattern recognition in the resulting electrocardiogram, so as to identify certain patterns and be able to provide corresponding information with respect to a stimulation outcome.

In one variant, the program prompts the processor to use a width of the QRS complex in the electrocardiogram, or an analysis criterion correlated with this QRS complex width, for the classification of the excitation state. The reason is that it is possible to determine from the width of the QRS complex whether a selective His bundle stimulation or a non-selective His bundle stimulation has taken place.

In one variant, the program prompts the processor to use a time delay between when the cardiac stimulation was carried out, on the one hand, and when the cardiac electrical signal was detected, on the other hand, for the classification of the excitation state. In other words, in this variant, the time that has passed between the stimulation and the detected cardiac excitation is taken into consideration so as to be able to appropriately classify the excitation state. The reason is that the different cardiac excitation states propagate at varying velocities. The point in time at which a cardiac excitation after prior stimulation can be detected is thus, at times, an important criterion to be able to assign the detected excitation to a particular class.

In one variant, the program prompts the processor to use at least one signal curve of the electrocardiogram for classifying the excitation state, wherein, in this variant, the electrocardiogram is a unipolar electrocardiogram from a pole of the electrode of the stimulation unit against a large-surface-area body electrode (for example, a housing of the system for stimulating the heart) of the particular patient.

One aspect of the present invention relates to a computer program product including computer-readable code, which prompts a processor to carry out the steps described hereafter when the code is being executed on the processor.

First, a cardiac stimulation is carried out by way of a stimulation unit.

Thereafter, a cardiac electrical signal is detected by way of a detection unit. This signal was generated by a cardiac excitation as a result of the cardiac stimulation carried out beforehand.

Thereafter, the excitation state of the heart stimulated by the cardiac stimulation is ascertained based on the detected electrical signal.

Now, the excitation state is classified into one of at least three classes. The at least three classes include a first class representative of a first excitation state, a second class representative of a second excitation state, which is different from the first excitation state, and a third class representative of a successful stimulation without a detectable excitation state.

Subsequently, an automatic adaptation of at least one control parameter of an implantable system for stimulating a human or an animal heart is carried out. This automatic adaptation takes place as a function of the classification that was carried out beforehand. The control parameter is a His bundle stimulation energy, a His bundle stimulation vector, an operating mode of the system used for stimulation, at least one parameter of a timer of the system, or a stimulation control parameter suitable for cardiac resynchronization therapy.

Another illustrative example which, however, is not part of the present invention relates to a medical method for treating a human patient or an animal patient in need of such treatment. This method is carried out by way of an implantable system for stimulating the heart of the patient. The system comprises a processor, a memory unit, a stimulation unit for stimulating the His bundle of the heart of the patient, and a detection unit for detecting an electrical signal of the heart of the patient. The method comprises the steps described hereafter.

First, a cardiac stimulation of the heart of the patient is carried out by way of the stimulation unit.

Thereafter, a cardiac electrical signal is detected by way of the detection unit. The cardiac electrical signal was triggered or generated by the cardiac stimulation that was carried out beforehand.

Then, an excitation state of the heart of the patient stimulated by the cardiac stimulation is ascertained. This ascertainment takes place based on the previously detected electrical signal.

Now, the ascertained excitation state is classified into one of at least three classes. These at least three classes include a first class for a first excitation state, a second class for a second excitation state, and a third class for an unsuccessful stimulation without a detectable excitation state. The second excitation state differs from the first excitation state.

After the classification, at least one control parameter of the system is automatically adapted as a function of the classification that was carried out. The control parameter can be a His bundle stimulation energy, a His bundle stimulation vector, an operating mode of the implantable system, at least one parameter of a timer of the system, or a stimulation control parameter suitable for a cardiac resynchronization therapy.

One aspect of the present invention relates to an analysis device for supporting the implantation of a system for stimulating the human or animal heart, having the features described hereafter. Such an analysis device comprises a processor and a memory unit.

According to the invention, the memory unit includes a computer-readable program, which prompts the processor to carry out the steps described hereafter when the program is being executed on the processor.

First, an electrocardiogram of a human heart or an animal heart into which a system for stimulating this heart is implanted is received. The electrocardiogram can be an electrocardiogram of any arbitrary type. Intracardiac electrograms are particularly suitable electrocardiograms.

Thereafter, an automatic identification of signals of the electrocardiogram that are caused by a His bundle stimulation is carried out. Each of these signals appears between an atrial signal and a ventricular signal.

According to advantageous embodiments of the present invention, signals of the electrocardiogram that are triggered by a His bundle stimulation can be identified by the following steps:
a) preprocessing the ECG signal by amplification, attenuation, filter, differentiation, smoothing, averaging, phase shift and/or offset correction; and
b) evaluating the ECG signal morphology to identify His bundle-typical signals. The His signal appears after an atrial signal and before a ventricular signal; according to embodiments of the invention, it is thus possible to identify the HIS signal between these. According to further aspects of the invention, a discrimination between the atrial and ventricular signals can be carried out by evaluating the signal amplitudes and ratios with respect to one another. With a suitable His position, the amplitude of the atrial signal is smaller than the amplitude of the His signal, and the amplitude of the ventricular signal is considerably greater (for example, 2 to 10 times) than the amplitude of the His signal.

Thereafter, the previously identified signals are marked in the received electrocardiogram.

The electrocardiogram thus marked is then output on an output device. The output device can be a component of the analysis device or can be a separate output device to which the analysis device is connected.

The marking that is carried out can be made visually or acoustically, for example. An optical marking can be achieved particularly easily, for example, by highlighting the identified His bundle-specific signals in color. This makes it significantly easier for a user of the analysis device to identify His bundle-specific signals within the electrocardiogram, and to correctly position an electrode provided for His bundle stimulation within a human heart or an animal heart based on the quality or characteristics of these His bundle-specific signals.

As a result of the use of such an analysis device, it is no longer necessary to carry out an electrophysiological examination, using an electrophysiological measuring station, during the implantation of a cardiac pacemaker or another system for stimulating the human or animal heart. Rather, such a separate examination is not required. Instead, it is possible, due to the use of the analysis device claimed according to the invention, to check directly during the implantation how well, that is to what extent, the His bundle of the particular heart is contacted by the corresponding electrode, and whether sufficient stimulation of the His bundle is ensured with the selected positioning of the electrode.

The output device can be a monitor or a combination of a monitor and a speaker, for example. As an alternative, the output device can also be a printer, which is suitable for printing a marked electrocardiogram on paper or another medium.

The analysis device can also be referred to as a pace-sense analyzer (PSA).

In one variant, the analysis device can be designed in the form of a separate device. In another variant, the analysis device is part of a system for stimulating the human or animal heart. In such a case, it may also be referred to as an analysis module. It is provided in this variant that the analysis device does not comprise a separate processor and does not comprise a separate memory unit, but resorts to a processor and a memory unit of the system for stimulating the human or animal heart. In this variant, the additional option of analyzing a correct electrode positioning can thus be implemented without additional apparatus-related complexity. This considerably simplifies the implantation of cardiac pacemakers suitable for His bundle stimulation.

In one variant, the analysis device comprises a stimulation unit for stimulating the His bundle of a human heart or an animal heart, and a detection unit for detecting an electrical signal of the same heart. In this variant, the program prompts the processor to carry out the steps described hereafter when the program is being executed on the processor.

First, a cardiac stimulation is carried out by way of the stimulation unit. This cardiac stimulation is suitable for ascertaining the stimulus threshold of the His bundle.

Thereafter, a cardiac electrical signal is detected, which was generated by the prior cardiac excitation as a result of the cardiac stimulation that was carried out. The detection unit is used for this purpose.

Thereafter, an excitation state of the heart stimulated by the cardiac stimulation is ascertained based on the detected electrical signal.

This excitation state is then classified into one of at least three classes. These at least three classes include a first class, which indicates a first excitation state of the heart. These further include a second class, which indicates a second excitation state of the heart different from the first excitation state. Finally, these include a third class, which indicates an unsuccessful stimulation without a detectable excitation state.

As a result of such a classification of a detected excitation state, it is particularly easy to identify whether sufficient contacting of the His bundle by way of an electrode of a cardiac pacemaker or of another system for stimulating the heart was achieved.

In one variant, suitable stimulation amplitudes of the pulses delivered by the stimulation unit for ascertaining the stimulus threshold of the His bundle are in a range of 12 V to 30 V, in particular 15 V to 25 V, and in particular 20 V to 22 V.

Suitable pulse widths of the pulses delivered by the stimulation unit for ascertaining the stimulus threshold of the His bundle are in a range between 1 ms and 15 ms, in particular between 2 ms and 12 ms, in particular between 3 ms and 10 ms, in particular between 4 ms and 9 ms, and in particular between 5 ms and 8 ms.

As was already mentioned, one of the at least three classes is a class that is provided for an unsuccessful stimulation without a detectable excitation state. In one variant, the remaining classes of the at least three classes (that is, the first class, the second class and an optionally provided further class) are selected from the classes described hereafter. One class relates to an excitation state of the heart for which only a stimulation of the His bundle of the stimulated heart has taken place. This means that an excitation state in which a selective stimulation of the His bundle has taken place falls into this class. Another class relates to an excitation state for which both a stimulation of the His bundle of the heart and a stimulation of the ventricular myocardium have taken place. This class thus relates to an excitation state that is based on non-selective stimulation. One class relates to an excitation state for which only a stimulation of the ventricular myocardium of the stimulated heart has taken place. This means that this excitation state is based on a stimulation in which the His bundle was not captured. This may be referred to as a purely ventricular excitation state. One class relates to an excitation state in which a His bundle stimulation and a stimulation of left ventricular conduction pathways have taken place. Another class relates to an excitation state in which a His bundle stimulation without stimulation of left ventricular conduction pathways has taken place. Such a stimulation is also referred to as a right bundle branch block (RBBB).

The classification of the excitation state into one of the predefined classes automatically implies a classification of the stimulation underlying the excitation state into such a class.

In one variant, the program prompts the processor to carry out an automatic stimulation threshold test (automated threshold monitoring, ATM). Within the scope of such a stimulation threshold test, a stimulus threshold for a successful His bundle stimulation is determined. A selective or non-selective His bundle stimulation is considered successful, whereas a stimulation in which the His bundle is not captured or which only results in ventricular stimulation is not considered successful.

In one variant, the program prompts the processor to classify each cardiac stimulation carried out by way of the stimulation unit. For this purpose, for example, active capture control (ACC) can be employed, optionally using a beat-to-beat algorithm.

In one variant, the program prompts the processor to use a width of the QRS complex in the electrocardiogram, or an analysis criterion correlated with this QRS complex width, for the classification of the excitation state. The reason is that it is possible to determine from the width of the QRS complex whether a selective His bundle stimulation or a non-selective His bundle stimulation has taken place.

In one variant, the program prompts the processor to use a time delay between when the cardiac stimulation was carried out, on the one hand, and when the cardiac electrical signal was detected, on the other hand, for the classification of the excitation state. In other words, in this variant, the time that has passed between the stimulation and the detected cardiac excitation is taken into consideration so as to be able to appropriately classify the excitation state. The reason is that the different cardiac excitation states propagate at varying velocities. The point in time at which a cardiac excitation after prior stimulation can be detected is thus, at times, an important criterion to be able to assign the detected excitation to a particular class.

In one variant, the program prompts the processor to use a classification of the excitation state of at least one signal curve of the electrocardiogram, wherein, in this variant, the electrocardiogram is a unipolar electrocardiogram from a pole of the electrode of the stimulation unit against a large-surface-area body electrode of the particular patient.

In one variant, the analysis device is equipped with a detection unit. This detection unit can, in particular, be particularly suitable for detecting His bundle-specific excitations pulses of the heart, or be specifically designed and configured to do so.

In one variant, the detection unit comprises a low-pass filter for this purpose, which is particularly suitable for signals generated by an excitation of the His bundle. Such a low-pass filter has a cut-off frequency of at least 100 kHz (that is, 100 kHz or greater), in particular at least 10 kHz, in particular at least 1 kHz, in particular at least 500 Hz, in particular at least 200 Hz, and most particularly at least 100 Hz. In one variant, such a low-pass filter has a cut-off frequency in a range between 100 Hz and 100 kHz, in particular between 200 Hz and 10 kHz, and in particular between 500 Hz and 1 kHz. Such a low-pass filter essentially allows signal components having a frequency below the cut-off frequency to pass unimpaired. In contrast, signal components having a frequency greater than the cut-off frequency are weakened or attenuated.

The particular suitability of the detection unit for detecting His bundle-specific pulses, or the design and configuration of the detection unit for this purpose, can also be implemented by a sampling rate that, in one variant, is at least 500 Hz, in particular at least 1 kHz, in particular at least 2 kHz, in particular at least 5 kHz, in particular at least 10 kHz, in particular at least 100 kHz, and in particular at least 1 MHz. In one variant, the sampling rate of the detection unit is in a frequency range between 500 Hz and 1 MHz, in particular between 1 kHz and 100 kHz, and in particular between 2 kHz and 10 kHz, and in particular between 3 kHz and 5 kHz.

In one variant, the detection unit has a sensitivity in a range between 0.01 mV and 0.25 mV, in particular between 0.05 mV and 0.2 mV, and in particular between 0.1 mV and 0.15 mV. This kind of sensitivity of the detection unit is particularly well-suited for being able to detect His bundle-specific electrical cardiac signals.

In one variant, the program prompts the processor to use a morphological analysis criterion from the electrocardiogram for automatically identifying the signals caused by a His bundle stimulation. In this way, it is possible, for example, to carry out a pattern recognition in the resulting electrocardiogram so as to identify certain patterns and thus enable the identification of His bundle-specific signals.

In one variant, the analysis device comprises a first stimulation unit for stimulating the His bundle of a human heart or an animal heart, a second stimulation unit for stimulating a cardiac region of the same heart different from the His bundle, a first detection unit and a second detection unit, which are each suitable for detecting an electrical signal of the same heart. In particular, an electrode of the second stimulation unit is provided to be implanted in an atrial or ventricular implantation site so as to serve as a conventional cardiac electrode for carrying out atrial or ventricular stimulation (that is, no stimulation of the His bundle). In particular, the first detection unit is provided to detect a stimulation of the His bundle of the heart. In contrast, the second detection unit is provided to detect a stimulation of a cardiac region by the second stimulation unit. If the second stimulation unit is provided for stimulating an atrium of the heart, the second detection unit is, in particular, an atrial detection unit. If the second stimulation unit is provided for stimulating a ventricular cardiac region, the second detection unit is, in particular, a ventricular detection unit. In this variant, it is thus not only possible to stimulate the His bundle of a heart and to detect the corresponding stimulation, but also to carry out atrial and/or ventricular stimulation, with subsequent detection of corresponding cardiac signals.

In one variant, the program prompts the processor to trigger a stimulation of the His bundle by way of the first stimulation unit upon detection of an intrinsic excitation of one of the two atria of the stimulated heart by the first detection unit or the second detection unit. By providing multiple stimulation units and multiple detection units, it is thus possible to trigger a stimulation of the His bundle as a function of other cardiac stimulations. This allows particularly effective stimulation of the His bundle to be achieved, whereby a positive influence on the cardiac signals, generated by a corresponding His bundle stimulation, and the subsequent detection thereof can be achieved.

In one variant, the analysis device is designed as a single channel stimulator or as part of such a single channel stimulator. Such a single channel stimulator comprises only one terminal for a stimulation and detection electrode. The stimulation and detection electrode forms part of the stimulation unit and of the detection unit in the process. As a result, it may also be referred to as a His bundle stimulation and detection electrode or as a His bundle stimulation and detection channel. The detection unit is not only provided and configured for detecting His bundle-specific cardiac signals of the heart to be stimulated. Rather, it is also provided and configured for detecting an intrinsic excitation of one of the two atria of the heart to be stimulated. Signals resulting from such an intrinsic excitation of one of the two atria are also referred to as a P wave. When the detection unit is provided and configured for detecting such atrial signals, the stimulation of the His bundle of the heart to be stimulated can be triggered or carried out particularly easily by the stimulation unit as a function of these intrinsic atrial signals.

In one variant, the analysis device is designed as a two-channel stimulator or as part of such a two-channel stimulator. The system then comprises a first terminal for a His bundle stimulation and detection electrode (or for a His bundle stimulation and detection channel), and a second terminal, which can be designed as an atrial stimulation and detection channel, or as a ventricular stimulation and detection channel, for connecting a corresponding atrial stimulation and detection electrode or a ventricular stimulation and detection electrode. In such a variant, the system typically comprises a second stimulation unit and a second detection unit, to which the atrial or ventricular stimulation and detection electrode is then assigned, in addition to the stimulation unit and the detection unit.

In one variant, the analysis device is designed as a three-channel stimulator or as part of such a three-channel stimulator. Such a three-channel stimulator comprises a first terminal for a His bundle stimulation and detection channel (or for a His bundle stimulation and detection electrode), a second terminal for an atrial stimulation and detection channel (or for an atrial stimulation and detection electrode), and a third terminal for a ventricular stimulation and detection channel (or for a ventricular stimulation and detection electrode). Such a three-channel stimulator can be used both to carry out His bundle-specific stimulations and detect His bundle-specific signals, and to carry out atrial and ventricular stimulations and detect atrial and ventricular signals particularly easily.

In one variant, the analysis device is designed as a four-channel stimulator or as part of such a four-channel stimulator. Such a four-channel stimulator comprises a first terminal for a His bundle stimulation and detection channel (or for a His bundle stimulation and detection electrode), a second terminal for an atrial stimulation and detection channel (or for an atrial stimulation and detection electrode), a third terminal for a first ventricular stimulation and detection channel (or a first ventricular stimulation and detection electrode), and a fourth terminal for a second ventricular stimulation and detection channel (or for a second ventricular stimulation and detection electrode). Such a four-channel stimulator can be used, for example, to stimulate both ventricles of a human heart or an animal heart simultaneously. Similarly, it is possible to detect signals from both ventricles simultaneously. Furthermore, the His bundle-specific stimulation and detection already described in connection with the preceding variants, and atrial stimulation and detection, are possible.

In another variant, the analysis device is designed as a five-channel stimulator or as part of such a five-channel stimulator. Such a five-channel stimulator comprises a total of five terminals. A first terminal is provided for a His bundle stimulation and detection channel (or for a His bundle stimulation and detection electrode). A second terminal is provided for an atrial stimulation and detection channel (or for an atrial stimulation and detection electrode). A third terminal is used to connect a first ventricular stimulation and detection channel (or a first ventricular stimulation and detection electrode). A fourth terminal is provided for a second ventricular stimulation and detection channel (or for a second ventricular stimulation and detection electrode). Finally, a fifth terminal is provided for a third ventricular stimulation and detection channel (or for a third ventricular stimulation and detection electrode). The implantable system can thus be used as a stimulator, for example, which is configured for cardiac resynchronization (CRT) (CRT stimulator) and has a quadripolar left ventricular channel. As an alternative, the implantable system can enable multipolar stimulation (known as multipole pacing), using a His bundle-specific stimulation channel.

In one variant, the analysis device comprises a device for automatic cardioversion and/or defibrillation of the heart or is part of such a device. It is then equipped with appropriate terminals so as to connect this device to suitable cardiac regions.

In one variant, the program prompts the processor to carry out a signal quality check (SQC) prior to ascertaining the excitation state. Such a signal quality check makes it possible to establish whether the signals provided for the ascertainment of the excitation state are suitable for the evaluation. This suitability can be derived, for example, from the absolute signal intensity or from a signal-to-noise ratio.

If the signal quality check shows that the quality of the signals provided for the ascertainment of the excitation state is not sufficient, the provided classification can be at least partially temporarily deactivated. As an alternative, it is also possible to appropriately identify the resultant classification result, that is, to indicate that this result was obtained based on signals that did not pass the signal quality check that was carried out. The corresponding classification is thus identified as being less reliable than other classifications.

In one variant, the program prompts the processor to automatically adapt a signal processing parameter of the system as a function of the signal quality check that was carried out. This may, for example, be an amplification, an attenuation, a filtering, a differentiation, a smoothing, an averaging, a phase shift or an offset correction of the measured signals, so as to be able to achieve a better evaluation of these signals. In this way, it becomes possible to amplify signals that may not pass, or just barely pass, the signal quality check or to ensure that these stand out against the background, so that the quality is nonetheless sufficient, that is, that the signal quality check is passed, thereby enabling a reliable evaluation of the corresponding signals.

One aspect of the present invention relates to a computer program product including computer-readable code, which prompts a processor to carry out the steps described hereafter when the code is being executed on the processor.

First, an electrocardiogram of a human heart or an animal heart into which a system for stimulating this heart is implanted is received.

Thereafter, an automatic identification of signals of the electrocardiogram that are caused by a His bundle stimulation is carried out. Each of these signals appears between an atrial signal and a ventricular signal.

Thereafter, the previously identified signals are marked in the received electrocardiogram.

Subsequently, the electrocardiogram thus marked is output on a suitable output device.

Such a computer program product is used to implement, in the form of software, those method steps that are also carried out by the analysis device claimed according to the invention. Such an analysis of cardiac signals for supporting the implantation of a system for stimulating the human or animal heart can thus be implemented as hardware (in the form of the analysis device claimed according to the invention) or as software (in the form of the computer program product claimed according to the invention).

An illustrative example which, however, is not part of the present invention relates to a method for evaluating data obtained during an implantation of a system for stimulating the human or animal heart. This method is purely a data evaluation method, which can be carried out without further interaction with a human or an animal body.

An electrocardiogram of a human heart or an animal heart into which a system for stimulating this heart is implanted is received for this data evaluation method.

Thereafter, signals of the electrocardiogram caused by a His bundle stimulation are automatically identified.

The signals thus identified are marked in the received electrocardiogram.

The electrocardiogram thus marked is then output on a suitable output device.

Another illustrative example which, however, is not part of the present invention relates to a method for implanting a system for stimulating the human or animal heart. This implantation is carried out in a patient in need of such a system. The implantation method comprises the steps described hereafter.

First, a region of the heart of the patient in which the His bundle of the heart is presumed to be located is contacted with an electrode. This electrode is part of a stimulation unit for stimulating the His bundle of a human heart or an animal heart.

Thereafter, a stimulation of the His bundle by way of the stimulation unit is carried out.

Thereupon, an electrocardiogram is detected by way of a detection unit. If the prior His bundle stimulation by way of the stimulation unit was successful, His bundle-specific signals can be found in this electrocardiogram.

These His bundle-specific signals are now automatically identified in the electrocardiogram.

The previously identified signals are marked in the received electrocardiogram. In this way, a marked electrocardiogram is obtained.

The marked electrocardiogram is output on an output device.

The position of the electrode can now be adapted as a function of the marked signals. Such an adaptation is not necessary if the His bundle-specific signals marked in the electrocardiogram already correspond to a predefined or predefinable quality criterion. If the marked signals are sufficiently large, for example, this indicates good contacting of the His bundle by way of the electrode. An adaptation of the position of the electrode can then be dispensed with.

If, in contrast, the signals do not yet indicate sufficient contacting of the His bundle (either because these are too weak overall or because they appear to be too weak or not pronounced enough compared to other cardiac signals), the position of the electrode is adapted. The steps of carrying out a stimulation of the His bundle, detecting an electrocardiogram, automatically identifying His bundle-specific signals in the electrocardiogram, marking these identified signals, and outputting the electrocardiogram thus marked are then repeated until the marked signals correspond to a selectable criterion. This selectable criterion can be the aforementioned predefined or predefinable quality criterion. For example, this criterion can be a qualitative or quantitative criterion of the detected and automatically identified His bundle-specific signals. An absolute value or a relative value of the corresponding signals can be used to check as to whether or not the criterion is satisfied.

If the selectable criterion is satisfied, that is, if, for example, sufficiently large or sufficiently pronounced His bundle-specific signals, or His bundle-specific signals that, overall, are sufficient compared to other cardiac signals, have been identified and marked in the electrocardiogram, the electrode is implanted in the most recently selected position. The reason is that this position is then suitable for sufficiently stimulating the His bundle of the heart.

All variants and alternative embodiments described in connection with the various implantable systems can be arbitrarily combined with one another and applied to the respective other systems. Similarly, they can also be applied in arbitrary combination to the described analysis unit, the described methods, and the described computer program products. The various variants of the analysis unit can further be arbitrarily combined with one another and applied to the different implantable systems, and can, analogously, also be applied to the described methods and the described computer program products. The described variants of the methods can further be arbitrarily combined with one another and applied to the respective other methods and to the computer program products and the systems as well as to the analysis unit. Similarly, the described variants of the computer program products can be arbitrarily combined with one another and applied to the respective other computer program products and to the described methods and the described systems, as well as to the analysis unit.

Further details of aspects of the invention are described in greater detail hereafter in connection with exemplary embodiments and drawings. In the drawings:
- FIG. 1: shows a block diagram of an exemplary embodiment of an implantable system for stimulating the human heart or animal heart;
- FIG. 2: shows a schematic representation of an exemplary embodiment of an implantable system for stimulation of the human or animal heart;
- FIG. 3: shows a block diagram of an exemplary embodiment of an implantable system for stimulating the human or animal heart, which carries out stimulation outcome monitoring during operation;
- FIG. 4: shows a simplified block diagram of an exemplary embodiment of an analysis device;
- FIG. 5: shows an exemplary embodiment of a representation output by the analysis device from FIG. 4; and
- FIG. 6: shows a block diagram of an exemplary embodiment of an implantable system for stimulating the human or animal heart.

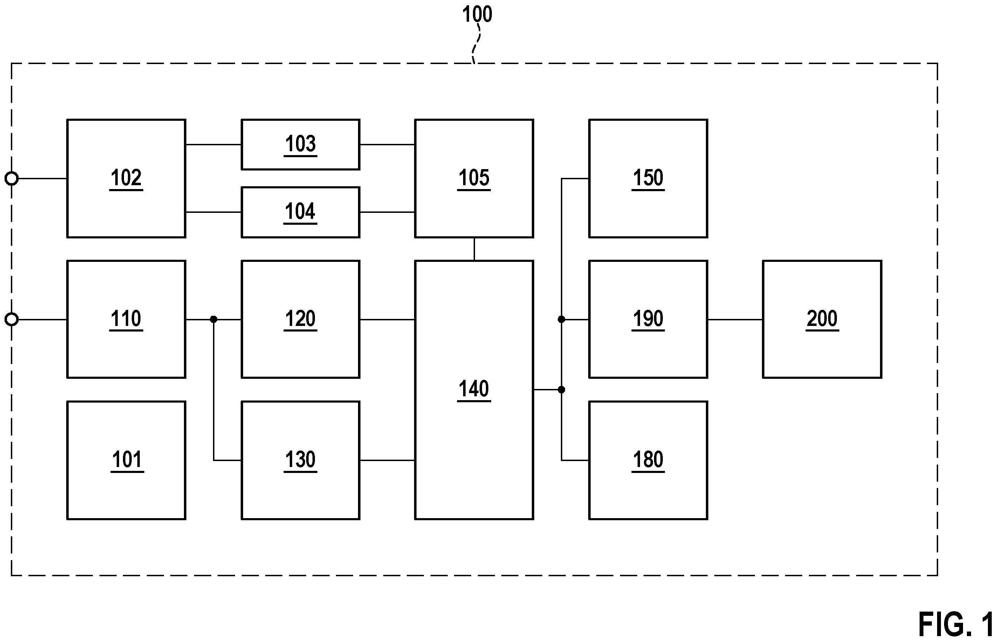

FIG. 1 shows a block diagram of an exemplary embodiment of a cardiac pacemaker 100, which is used as an implantable system for stimulating the human or animal heart. The cardiac pacemaker 100 comprises a power source 101 and a first electrode terminal 102, which serves as a first stimulation output. A first stimulation unit 103, from which stimulation pulses can be conducted through the first electrode terminal 102 to a first electrode, is connected to the electrode terminal 102. Moreover, a first detection unit 104 is connected to the first electrode terminal 102. The first stimulation unit 103 and the first detection unit 104 act as conventional detection and stimulation stages of the cardiac pacemaker 100.

So as to ensure a defined delivery of stimulation pulses, in terms of time, by the first stimulation unit 103 and to adapt the corresponding stimulation pulses to the signals of a heart detected by the first detection unit 104, the cardiac pacemaker 100 further comprises a first timer 105, which likewise functions in a conventional manner.

In addition, the cardiac pacemaker 100 comprises a second electrode terminal 110, which serves as a second stimulation output. It is connected to a second stimulation unit 120 and a second detection unit 130. The second stimulation unit is specifically designed and configured to carry out a stimulation of the His bundle of the heart to be treated. The second detection unit 130 is specifically designed and configured to detect an electrical signal of the His bundle of this heart.

Both the second stimulation unit 120 and the second detection unit 130 are connected to a second timer 140. The timer is additionally operatively connected to the first timer 105. It is possible, by way of the timer 140, to synchronize the stimulation pulses to be delivered by the second stimulation unit 120 with the stimulation pulses being delivered by the first stimulation unit 103. In this way, the His bundle of the heart can be stimulated at a point in time at which the His bundle is particularly receptive to such a stimulation and which is an obvious choice, in a physiologically meaningful manner, for restoring a natural cardiac rhythm. The second timer 140 also opens up the option of achieving synchronization of the delivery, in terms of time, of stimulation pulses delivered by the first stimulation unit 103 with stimulation pulses delivered by the second stimulation unit 120 via signals detected by the second detection unit 130.

A His bundle marker channel 150, a His bundle stimulation threshold test unit, which serves as a first stimulation threshold test device, and a His bundle diagnostic memory 190 are arranged downstream of the second timer 140.

It is possible, by way of the His bundle marker channel 150, to read out an electrocardiogram (ECG) or an intracardiac electrogram (IEGM) from the cardiac pacemaker 100, wherein the ECG or the IEGM is provided with markings that are specific to a His bundle stimulation by way of the second stimulation unit 120 and/or specific to the detection of a signal of the His bundle by way of the second detection unit 130. In this way, it is possible to retrieve ECGs or IEGMs provided with His bundle-specific information from the cardiac pacemaker 100 via the His bundle marker channel 150.

The His bundle stimulation threshold test unit 180 is used to determine a stimulus threshold of the His bundle before a corresponding stimulation of the His bundle is carried out by way of the second stimulation unit 120. In this way, it is possible, at all times, to provide a sufficiently strong stimulation pulse by way of the second stimulation unit 120, without having to expend more energy than necessary. The His bundle stimulation threshold test unit 180 is thus used, on the one hand, to ensure that the stimulation pulses delivered by the second stimulation unit 120 are strong enough to achieve an excitation of the His bundle, but, on the other hand, that the stimulation pulses delivered by the second stimulation unit 120 have the lowest possible energy, so that the load posed by the second stimulation unit 120 on the power source 101 of the cardiac pacemaker 100 is minimized.

Events that relate to the His bundle stimulation or the His bundle activity are stored in the His bundle diagnostic memory 190, which can be configured as a memory area of a larger memory unit, for example. The His bundle diagnostic memory is used, for example, to record stimulation pulses delivered by the second stimulation unit 120, and additionally to also record data detected by the second detection unit 130 with respect to the His bundle of the heart to be treated.

A His bundle remote monitoring and programming unit 200, which can be used to read out the His bundle diagnostic memory 190, is assigned to the His bundle diagnostic memory 190. In addition, the second stimulation unit 120 and/or the second detection unit 130 can be monitored by way of the His bundle remote monitoring and programming unit 200. It is further possible to adapt the His bundle stimulation to be delivered by the second stimulation unit 120 by way of the His bundle remote monitoring and programming unit 200. This His bundle remote monitoring and programming unit 200 thus allows access to specific components of the cardiac pacemaker 100, wherein it is made possible to both read out data and write data.

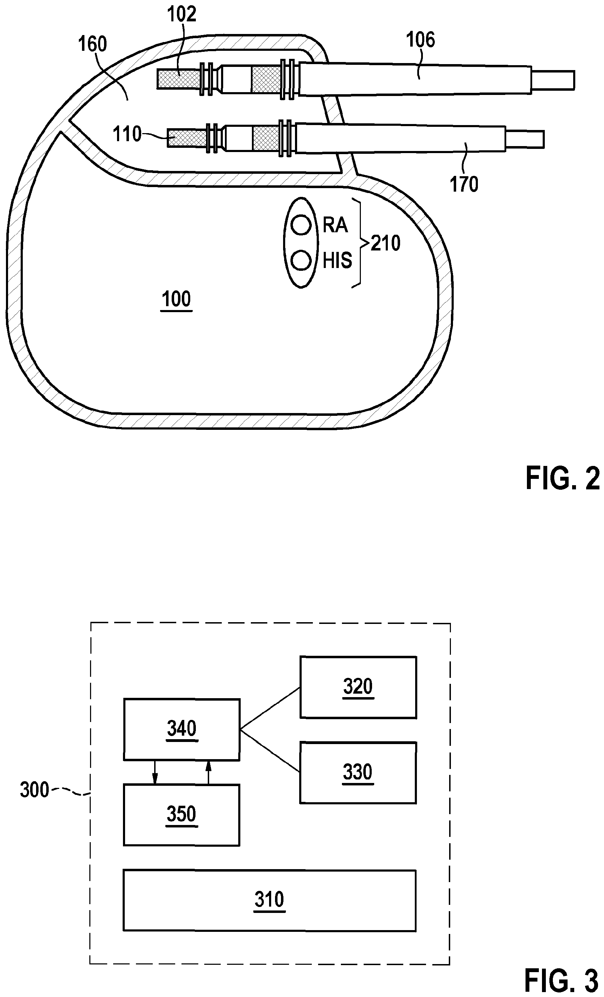

FIG. 2 shows a schematic side view of the cardiac pacemaker 100, of which the block diagram is shown in FIG. 1. Like elements are denoted by like reference numerals.

A header 160, in which the first electrode terminal 102 and the second electrode terminal 110 are formed, is apparent in the schematic illustration of FIG. 2. A first electrode 106 is plugged into the first electrode terminal 102, a second electrode 170 is plugged into the second electrode terminal 110. The first electrode 106 and the second electrode 107 are shown in sections in the illustration of FIG. 2.

The first electrode 106 is used to conventionally stimulate an arbitrary cardiac region of one of the ventricles. These are also used to detect electrical signals in one of these ventricles.

The second electrode 170 is used specifically to stimulate a His bundle and to detect His bundle-specific electrical signals. So as to enable easier use of the cardiac pacemaker 100 for a user, the second electrode 170 is identified as a His bundle electrode by the label "HIS." To ensure that this second electrode 170 is plugged into the correct electrode terminal, this being the second electrode terminal 110, this terminal is provided with the additional identification "HIS" in the header 160 of the cardiac pacemaker 100.

It can also be provided that the second electrode terminal 110 is configured to be structurally different from the first electrode terminal 102, so that it is not possible to plug the second electrode 170 (this being the His bundle electrode) into the first electrode terminal 102 in the first place.

In addition to a label such as "HIS," color coding of the second electrode 170 and/or the second electrode terminal 110, or of a corresponding region of the header 160, can be provided.

On the side, the cardiac pacemaker 100 further includes a marking 210, which schematically represents the first electrode terminal 100 and the second electrode terminal 110 and provides it with a corresponding identification. The marking 210 indicates that the first (upper) electrode terminal 102 is provided for connecting a first electrode 106 leading into the right atrium (RA), and the second (lower) electrode terminal 110 is provided for connecting a second electrode 170 leading to the His bundle. This additional marking 210 additionally facilitates the connection of the correct electrodes 106, 170 to the intended electrode terminals 102, 110 for a user of the cardiac pacemaker 100.

FIG. 3 shows a block diagram of an exemplary embodiment of a cardiac pacemaker 300, which is used as an implantable system for stimulating the human or animal heart. This cardiac pacemaker 300 carries out stimulation outcome monitoring and is able to automatically adapt an internal control parameter as a function of the stimulation outcome monitoring that was carried out.

The cardiac pacemaker comprises a power source 310 and a His bundle stimulation unit 320, which serves as a stimulation unit. Furthermore, a detection unit 330 is provided, which serves as a detection unit. The His bundle stimulation unit 320 and the detection unit 330 are operatively connected to a processor 340. This processor 340, in turn, can access a memory unit 350 and receive data from or send data to this memory unit 350.

A program which the processor 340 can use to carry out certain steps is stored in the memory unit 350. For example, the processor 340 prompts the His bundle stimulation unit 320 to carry out a cardiac stimulation (in particular of the His bundle) by way of a stimulation electrode, which is not shown in FIG. 3. Thereafter, the processor 340 prompts the detection unit 330 to detect a cardiac electrical signal from the previously stimulated heart. This signal is then used to ascertain an excitation state of the heart.

The excitation state is classified into one of at least three different classes. Thereafter, a specific control parameter of the cardiac pacemaker 300 is automatically adapted as a function of the classification that was carried out.

The steps of ascertaining the excitation state, of classifying the excitation state, and of automatically adapting at least one control parameter can be carried out in a stimulation outcome monitoring module, which can be implemented either as hardware or as software. In the exemplary embodiment of FIG. 3, this stimulation outcome monitoring module is implemented as software, so that it is not shown separately.

As a result of the automatic adaptation of at least one control parameter of the cardiac pacemaker 300, a therapeutic outcome of a previously carried out His bundle stimulation can be monitored in a particularly simple manner, wherein a particularly safe and effective therapy can be ensured by the automatic adaptation of a control parameter, even over an extended period of time.

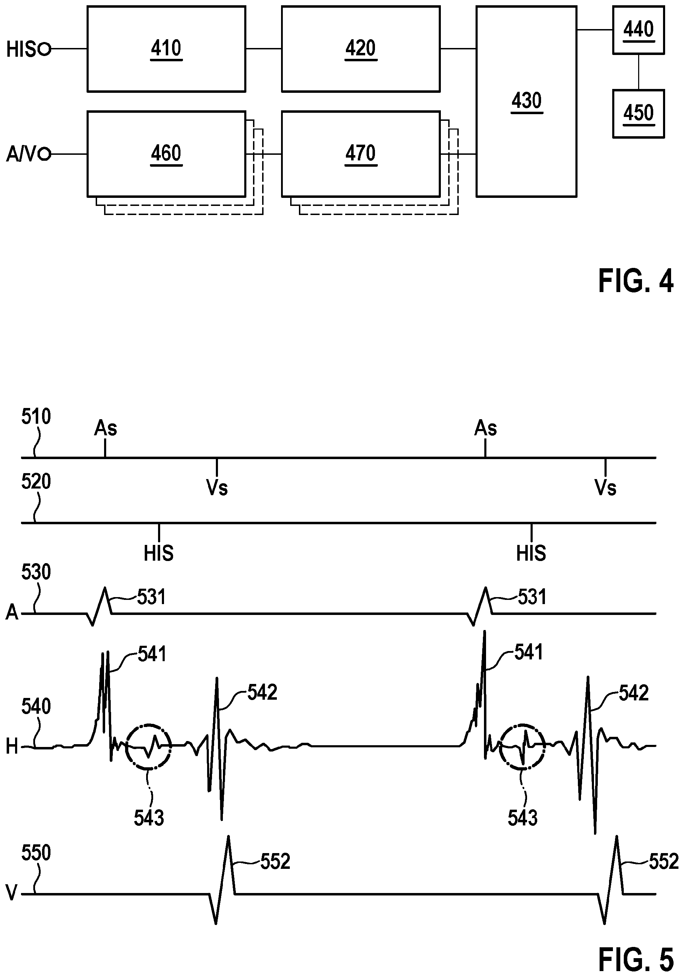

FIG. 4 shows a simplified block diagram of a His bundle pace-sense analyzer (His PSA), which serves as an analysis device. This His PSA comprises a His bundle stimulation unit 410, which includes an electrode interface by way of which a His bundle electrode can be connected to the His bundle stimulation unit 410. The His bundle stimulation unit 410 is used to deliver high energy stimulation, by which the His bundle of a human heart or an animal heart can be easily stimulated.

The His PSA further comprises a first detection unit 420, which can likewise directly access an electrode, by which a stimulation of the His bundle can be detected. The first detection unit 420 is provided and configured to record an electrocardiogram in the form of a broadband intracardiac electrogram (IEGM) and to subject it to a morphological signal analysis. Using a digital signal processor (DSP), which is an integral part of the first detection device 420, it is possible to identify and mark His bundle-specific signal morphologies within the captured electrocardiogram. The first detection unit 420 then forwards the further processed IEGM to a control and display unit 430, which serves as an output device for outputting the marked IEGM.

The control and display unit 430 is additionally connected to a processor 440, which, in turn, can access a memory unit 450. In this way, it is possible for the processor 440 to retrieve program information from the memory unit 450 and to transmit this information via the control and display unit 430 to the His bundle stimulation unit 410 and the first detection unit 420.

In addition, the His PSA comprises one or more further stimulation units 460, which are designed as conventional stimulation units and comprise conventional electrode interfaces for connecting atrial or ventricular electrodes. Conventional stimulation specifications of the corresponding electrodes can be provided in the process. In addition to the further stimulation units 460, one or more further detection units 470 are also provided, which are used to detect and evaluate atrial and ventricular cardiac signals. These further detection units 470 use conventional detection specifications for this purpose. Using these further detection units 470, it is possible to identify and mark ventricular and atrial signal in recorded electrocardiograms. The control and display unit 430 can thus display both His bundle-specific signals and atrial and/or ventricular signals in an electrocardiogram, such as an IEGM. The control and display unit 430 is able to represent different channels (in particular, an atrial channel, a ventricular channel, and a His bundle-specific channel) separately from one another.

This is shown schematically in FIG. 5 by way of example. For example, from top to bottom, FIG. 5 shows a first marker channel 510 for marking atrial signals As and ventricular signals Vs.

Beneath, a second marker channel 520 is shown, which is used to mark His bundle-specific signals HIS.

Only atrial signals 531 are schematically represented in an atrial IEGM channel 530.

In addition to atrial signals 541 and ventricular signals 542, His bundle-specific signals 543 are represented and separately marked in a His bundle-specific channel 540. This can typically be carried out by highlighting in color. In the illustration of FIG. 5, the His bundle-specific signals 543 are shown circled.

Finally, only ventricular signals 552 are schematically represented in a ventricular IEGM channel 550.

A user can have multiple channels 510 to 550 displayed simultaneously, or can have individual channels 510 to 550 displayed separately from other channels 510 to 550. In this way, it is possible to filter the information relevant for the particular problem for the user in a particularly simple manner. As a result of the automatic identification and separate marking of His bundle-specific signals 543, it is particularly easy to verify a correct positioning of a His bundle-specific stimulation electrode, and to optimize it with respect to the best possible contacting of the His bundle.

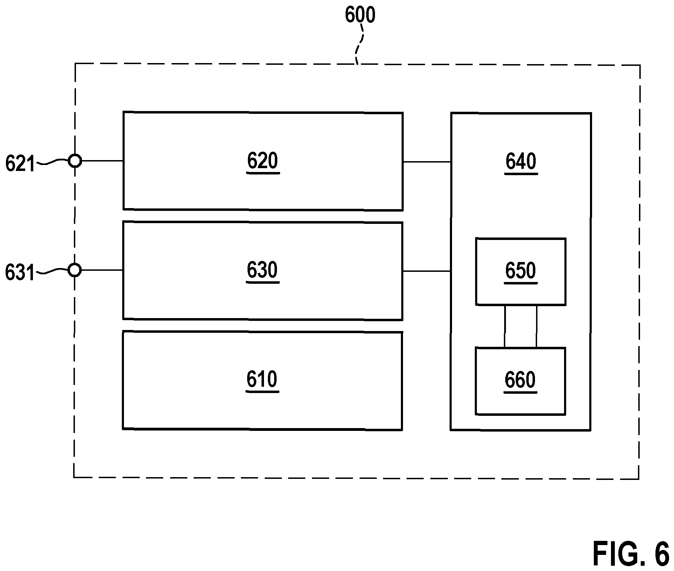

FIG. 6 shows a cardiac pacemaker 600, which is used as an implantable system for stimulating the human or animal heart. This cardiac pacemaker 600 is specifically provided and configured for treating an AV nodal reentry tachycardia (AVNRT).

The cardiac pacemaker 600 comprises a power source 610, a tachycardia identification and classification unit 620, which serves as a detection unit, and a His bundle stimulation unit 630, which serves as a stimulation unit. The tachycardia identification and classification unit 620 and the stimulation unit 630 are connected to a control unit 640. The control unit 640, in turn, is operatively connected to a processor 650, which can access a memory unit 660. The tachycardia identification and classification unit 620 comprises an electrode terminal 621 to which a first electrode can be connected. By way of this first electrode, the tachycardia identification and classification unit 620 is able to identify whether tachycardia is present in the heart of the patient wearing the cardiac pacemaker 600. The tachycardia identification and classification unit 620 is additionally able to identify and classify the type of tachycardia. In particular, an AV nodal reentry tachycardia can be distinguished by the tachycardia identification and classification unit 620 from other tachycardias.

When such an AV nodal reentry tachycardia is identified by the tachycardia identification and classification unit 620, the control unit 640 ensures that the stimulation unit 630 delivers a stimulation that is specifically suitable for stimulating the His bundle of the heart of the patient. For this purpose, the stimulation unit 630 comprises an electrode terminal 631, to which a second electrode, which is arranged in the his bundle or so close to the His bundle that a His bundle stimulation is possible by way of this electrode, is connected during operation of the cardiac pacemaker 600. The control unit 640 transmits the corresponding signal to the stimulation unit 630 after having received a corresponding command from the processor 650. A program which the processor 650 obtains from the memory unit 660 runs on the processor 650.

The individual components of the cardiac pacemaker 600 are supplied with the power necessary for operation from the power source 610.

The tachycardia identification and classification unit 620 uses ECG signals, received via the electrode terminal 621, for identifying and classifying a tachycardia, and in particular for identifying an AV nodal reentry tachycardia. The ECG signals can be derived from the atrium and/or the ventricle of the heart of the patient. In addition, it is possible to provide corresponding ECG signals directly via a His bundle electrode, for example via the second electrode, which is also connected to the second electrode terminal 631 of the stimulation unit 630.

So as to classify the established tachycardia, that is, so as to distinguish different tachycardias from one another, and, in particular, so as to identify an AV nodal reentry tachycardia, the tachycardia identification and classification unit 620 can resort to morphological analysis criteria within the provided electrocardiogram or, instead of such a morphological analysis of the electrocardiogram, can evaluate a chronological sequence of atrial and/or ventricular signals in the electrocardiogram. It is also possible to carry out a morphological analysis of a provided electrocardiogram, and to carry out an analysis of the chronological sequence of the signals present in this electrocardiogram.

This cardiac pacemaker 600 provides a new, device-based therapy for tachycardias, and in particular, for AV node reentry tachycardia. As a result, the treatment spectrum of active implants is enhanced.

## Claims

1. An implantable system for stimulating a human heart or an animal heart, comprising a processor (650), a memory unit (660), a stimulation unit (630) for stimulating a His bundle of a human heart or an animal heart, and a detection unit (620) for detecting an electrical signal of the same heart,
wherein
the memory unit (660) includes a computer-readable program, which prompts the processor (650) to carry out the following steps when the program is being executed on the processor (650):
a) detecting by way of the detection unit (620) whether a tachycardia is present in a human heart or an animal heart; and
b) when a tachycardia is present, carrying out a His bundle stimulation by way of the stimulation unit (630) using at least one stimulation pulse
having an amplitude in a range of 7.5 V to 30 V, and having a pulse width in a range of 1 ms to 15 ms,
wherein the program prompts the processor to classify a detected tachycardia into one of at least two classes, and
wherein the stimulation of the His bundle is triggered depending on the class into which the detected tachycardia is classified.

2. The implantable system according to claim 1, **characterized in that** a first of the at least two classes is provided for an AV nodal reentry tachycardia.

3. The implantable system according to any one of the preceding claims, **characterized in that** the program prompts the processor (650) to carry out the His bundle stimulation only if the tachycardia was identified as an AV nodal reentry tachycardia.

4. The implantable system according to any one of the preceding claims, **characterized in that** the program prompts the processor (650) to evaluate signals of an electrocardiogram so as to ascertain whether tachycardia is present.

5. The implantable system according to claim 4, **characterized in that** the electrocardiogram is an intracardiac electrogram or a far field electrocardiogram.

6. The implantable system according to claim 4 or 5, **characterized in that** the program prompts the processor (650) to evaluate a chronological sequence of atrial and ventricular signals in the electrocardiogram so as to ascertain whether tachycardia is present.

7. The implantable system according to any one of the preceding claims, **characterized in that** the program prompts the processor (650) to carry out the His bundle stimulation in the form of a pulse sequence comprising at least two pulses, a time delay between two pulses of the pulse sequence being smaller than a cycle length of the ascertained tachycardia.

8. The implantable system according to any one of claims 1 to 6, **characterized in that** the program prompts the processor (650) to carry out the His bundle stimulation in the form of a pulse sequence comprising at least two pulses, a time delay between two pulses of the pulse sequence being greater than a cycle length of the ascertained tachycardia.

9. The implantable system according to any one of claims 1 to 6, **characterized in that** the program prompts the processor (650) to carry out the His bundle stimulation by way of a single pulse.

10. A computer program product including computer-readable code, which prompts a processor (650) to carry out the following steps when the code is being executed on the processor (650):
a) detecting by way of a detection unit (620) whether a tachycardia is present in a human heart or an animal heart; and
b) when a tachycardia is present, carrying out a His bundle stimulation by way of a stimulation unit (630) using at least one stimulation pulse,
having an amplitude in a range of 7.5 V to 30 V, and having a pulse width in a range of 1 ms to 15 ms, wherein the program prompts the processor to classify a detected tachycardia into one of at least two classes, and
wherein the stimulation of the His bundle is triggered depending on the class into which the detected tachycardia is classified.

## Patentansprüche

1. Implantierbares System zum Stimulieren eines menschlichen Herzens oder eines Tierherzens, umfassend einen Prozessor (650), eine Speichereinheit (660), eine Stimulationseinheit (630) zum Stimulieren eines His-Bündels eines menschlichen Herzens oder Tierherzens und eine Detektionseinheit (620) zum Detektieren eines elektrischen Signals desselben Herzens ,
wobei
die Speichereinheit (660) ein computerlesbares Programm umfasst, das den Prozessor (650) veranlasst, die folgenden Schritte auszuführen, wenn das Programm auf dem Prozessor (650) ausgeführt wird:
a) Erkennen, mittels der Detektionseinheit (620), ob eine Tachykardie in einem menschlichen Herzen oder Tierherzen vorliegt;
b) wenn eine Tachykardie vorliegt, Durchführen einer His-Bündel-Stimulation mittels der Stimulationseinheit (630) unter Verwendung mindestens eines Stimulationsimpulses
mit einer Amplitude in einem Bereich von 7,5 V bis 30 V, und mit einer Pulsbreite in einem Bereich von 1 ms bis 15 ms,
wobei das Programm den Prozessor veranlasst, eine erkannte Tachykardie in eine von mindestens zwei Klassen einzustufen, und
wobei die Stimulation des His-Bündels abhängig von der Klasse, in welche die erkannte Tachykardie eingestuft wird, ausgelöst wird.

2. Implantierbares System nach Anspruch 1, **dadurch gekennzeichnet, dass** eine erste von den mindestens zwei Klassen für eine AV-Knoten-Reentry-Tachykardie vorgesehen ist.

3. Implantierbares System nach einem der vorstehenden Ansprüche, **dadurch gekennzeichnet, dass** das Programm den Prozessor (650) veranlasst, die His-Bündel-Stimulation nur dann auszuführen, wenn die Tachykardie als eine AV-Knoten-Reentry-Tachykardie identifiziert wurde.

4. Implantierbares System nach einem der vorstehenden Ansprüche, **dadurch gekennzeichnet, dass** das Programm den Prozessor (650) veranlasst, Signale eines Elektrokardiogramms auszuwerten, um festzustellen, ob eine Tachykardie vorliegt.

5. Implantierbares System nach Anspruch 4, **dadurch gekennzeichnet, dass** das Elektrokardiogramm ein intrakardiales Elektrokardiogramm oder ein Far-Field-Elektrokardiogramm ist.

6. Implantierbares System nach Anspruch 4 oder 5, **dadurch gekennzeichnet, dass** das Programm den Prozessor (650) veranlasst, eine chronologische Sequenz von Vorhof- und Ventrikel-Signalen im Elektrokardiogramm auszuwerten, um festzustellen, ob eine Tachykardie vorliegt.

7. Implantierbares System nach einem der vorstehenden Ansprüche, **dadurch gekennzeichnet, dass** das Programm den Prozessor (650) veranlasst, die His-Bündel-Stimulation in Form einer Impuls-Sequenz auszuführen, die mindestens zwei Impulse umfasst, wobei eine Zeitverzögerung zwischen zwei Impulsen der Impulssequenz kleiner als eine Zykluslänge der festgestellten Tachykardie ist.

8. Implantierbares System nach einem der Ansprüche 1 bis 6, **dadurch gekennzeichnet, dass** das Programm den Prozessor (650) veranlasst, die His-Bündel-Stimulation in Form einer Impuls-Sequenz auszuführen, die mindestens zwei Impulse umfasst, wobei eine Zeitverzögerung zwischen zwei Impulsen der Impulssequenz größer als eine Zykluslänge der festgestellten Tachykardie ist.

9. Implantierbares System nach einem der Ansprüche 1 bis 6, **dadurch gekennzeichnet, dass** das Programm den Prozessor (650) veranlasst, die His-Bündel-Stimulation in Form eines Einzelimpulses auszuführen.

10. Computerprogrammprodukt, computerlesbaren Code einschließend, das einen Prozessor (650) veranlasst, die folgenden Schritte auszuführen, wenn der Code im Prozessor (650) ausgeführt wird:
a) Erkennen, mittels einer Detektionseinheit (620), ob eine Tachykardie in einem menschlichen Herzen oder Tierherzen vorliegt; und
b) wenn eine Tachykardie vorliegt, Durchführen einer His-Bündel-Stimulation mittels einer Stimulationseinheit (630) unter Verwendung mindestens eines Stimulationsimpulses,
mit einer Amplitude in einem Bereich von 7,5 V bis 30 V und mit einer Pulsbreite in einem Bereich von 1 ms bis 15 ms, wobei das Programm den Prozessor veranlasst, eine erkannte Tachykardie in eine von mindestens zwei Klassen einzustufen, und
wobei die Stimulation des His-Bündels abhängig von der Klasse, in welche die erkannte Tachykardie eingestuft wird, ausgelöst wird.

## Revendications

1. Système implantable pour stimuler un coeur humain ou un coeur animal, comprenant un processeur (650), une unité de mémoire (660), une unité de stimulation (630) pour stimuler un faisceau de His d'un coeur humain ou d'un coeur animal, et une unité de détection (620) pour détecter un signal électrique du même coeur,
dans lequel
l'unité de mémoire (660) comprend un programme lisible par ordinateur, qui invite le processeur (650) à mettre en oeuvre les étapes suivantes lorsque le programme est exécuté sur le processeur (650) :
a) détecter au moyen de l'unité de détection (620) si une tachycardie est présente dans un coeur humain ou un coeur animal ; et
b) lorsqu'une tachycardie est présente, mettre en oeuvre une stimulation du faisceau de His au moyen de l'unité de stimulation (630) en utilisant au moins une impulsion de stimulation
ayant une amplitude dans une plage de 7,5 V à 30 V, et ayant une largeur d'impulsion dans une plage de 1 ms à 15 ms,
dans lequel le programme invite le processeur à classer une tachycardie détectée en une parmi au moins deux classes, et
dans lequel la stimulation du faisceau de His est déclenchée en fonction de la classe dans laquelle la tachycardie détectée est classée.

2. Système implantable selon la revendication 1, **caractérisé en ce que** une première des au moins deux classes est fournie pour une tachycardie par réentrée intranodale AV.

3. Système implantable selon l'une quelconque des revendications précédentes, **caractérisé en ce que** le programme invite le processeur (650) à mettre en oeuvre la stimulation du faisceau de His uniquement si la tachycardie a été identifiée comme une tachycardie par réentrée intranodale AV.

4. Système implantable selon l'une quelconque des revendications précédentes, **caractérisé en ce que** le programme invite le processeur (650) à évaluer des signaux d'un électrocardiogramme de manière à constater si une tachycardie est présente.

5. Système implantable selon la revendication 4, **caractérisé en ce que** l'électrocardiogramme est un électrogramme intracardiaque ou un électrocardiogramme de champ lointain.

6. Système implantable selon la revendication 4 ou 5, **caractérisé en ce que** le programme invite le processeur (650) à évaluer une séquence chronologique de signaux auriculaires et ventriculaires dans l'électrocardiogramme de manière à constater si une tachycardie est présente.

7. Système implantable selon l'une quelconque des revendications précédentes, **caractérisé en ce que** le programme invite le processeur (650) à mettre en oeuvre la stimulation du faisceau de His sous la forme d'une séquence d'impulsions comprenant au moins deux impulsions, un délai entre deux impulsions de la séquence d'impulsions étant inférieur à une longueur de cycle de la tachycardie constatée.

8. Système implantable selon l'une quelconque des revendications 1 à 6, **caractérisé en ce que** le programme invite le processeur (650) à mettre en oeuvre la stimulation du faisceau de His sous la forme d'une séquence d'impulsions comprenant au moins deux impulsions, un délai entre deux impulsions de la séquence d'impulsions étant supérieur à une longueur de cycle de la tachycardie constatée.

9. Système implantable selon l'une quelconque des revendications 1 à 6, **caractérisé en ce que** le programme invite le processeur (650) à mettre en oeuvre la stimulation du faisceau de His au moyen d'une impulsion unique.

10. Produit de programme informatique incluant un code lisible par ordinateur, qui invite un processeur (650) à mettre en oeuvre les étapes suivantes lorsque le code est exécuté sur le processeur (650) :
a) détecter au moyen d'une unité de détection (620) si une tachycardie est présente dans un coeur humain ou un coeur animal ; et
b) lorsqu'une tachycardie est présente, mettre en oeuvre une stimulation du faisceau de His au moyen d'une unité de stimulation (630) en utilisant au moins une impulsion de stimulation,
ayant une amplitude dans une plage de 7,5 V à 30 V, et ayant une largeur d'impulsion dans une plage de 1 ms à 15 ms, dans lequel le programme invite le processeur à classer une tachycardie détectée dans l'une parmi au moins deux classes, et
dans lequel la stimulation du faisceau de His est déclenchée en fonction de la classe dans laquelle la tachycardie détectée est classée.
